(19) Europäisches Patentamt
European Patent Office
Office européen des brevets

(11) **EP 2 152 881 B1**

(12) **FASCICULE DE BREVET EUROPEEN**

(45) Date de publication et mention
de la délivrance du brevet:
**23.04.2014 Bulletin 2014/17**

(21) Numéro de dépôt: **08805580.1**

(22) Date de dépôt: **16.05.2008**

(51) Int Cl.:
*C12N 15/31* (2006.01)    *C07K 7/06* (2006.01)
*C07K 14/195* (2006.01)    *C07K 7/04* (2006.01)
*A23L 1/305* (2006.01)    *A23K 1/16* (2006.01)
*A61K 38/04* (2006.01)    *A61K 35/74* (2006.01)
*C07K 14/33* (2006.01)    *A23L 1/30* (2006.01)

(86) Numéro de dépôt international:
**PCT/FR2008/000683**

(87) Numéro de publication internationale:
**WO 2008/152252 (18.12.2008 Gazette 2008/51)**

(54) **PEPTIDES RUMC PRÉSENTANT UNE ACTIVITÉ ANTIMICROBIENNE**

RUMC-PEPTIDE MIT ANTIMIKROBIELLER WIRKUNG

RUMC PEPTIDES WITH ANTIMICROBIAL ACTIVITY

(84) Etats contractants désignés:
**AT BE BG CH CY CZ DE DK EE ES FI FR GB GR HR HU IE IS IT LI LT LU LV MC MT NL NO PL PT RO SE SI SK TR**

(30) Priorité: **29.05.2007 FR 0703789**

(43) Date de publication de la demande:
**17.02.2010 Bulletin 2010/07**

(73) Titulaire: **Adisseo France S.A.S.**
**92160 Antony (FR)**

(72) Inventeurs:
• **CROST, Emmanuelle**
**NR2 3EA**
**Norwich**
**Norfolk (GB)**
• **FONS, Michel**
**F-13090 Aix en Provence (FR)**
• **GERAERT, Pierre-André**
**F-37210 Rochecorbon (FR)**

(74) Mandataire: **Delorme, Nicolas et al**
**Cabinet Germain & Maureau**
**BP 6153**
**69466 Lyon Cedex 06 (FR)**

(56) Documents cités:
• **DABARD J ET AL: "Ruminococcin A, a new lantibiotic produced by a Ruminococcus gnavus strain isolated from human feces" APPLIED AND ENVIRONMENTAL MICROBIOLOGY, vol. 67, no. 9, septembre 2001 (2001-09), pages 4111-4118, XP002464220 ISSN: 0099-2240**
• **GOMEZ ANA ET AL: "Trypsin mediates growth phase-dependent transcriptional regulation of genes involved in biosynthesis of ruminococcin A, a lantibiotic produced by a Ruminococcus gnavus strain from a human intestinal microbiota" JOURNAL OF BACTERIOLOGY, vol. 184, no. 1, janvier 2002 (2002-01), pages 18-28, XP002464221 ISSN: 0021-9193**

**Description**

L'invention concerne les peptides RumC1, RumC2 et RumC3 présentant une activité antimicrobienne, ainsi que les gènes codant pour ces peptides et isolés de *Ruminococcus gnavus* E1.

Certaines souches bactériennes ont la capacité de libérer des substances ayant un effet bactériostatique ou bactéricide sur leurs compétiteurs. Ces substances antimicrobiennes peuvent être de nature organique, par exemple acides organiques ou peroxyde d'hydrogène (Ross et al., Int. J. Food Microbiol. 79, 3-16, 2002), ou de nature peptidique. On distingue, en outre, les peptides antimicrobiens synthétisés par voie enzymatique qui forment la classe des antibiotiques (Mootz et al., Curr. Opin. Chem. Biol. 1, 543-551, 1997; Keating et al., Curr. Opin. Chem. Biol. 3, 598-606, 1999), et les peptides produits par la voie ribosomique qui forment la classe des bactériocines (Jacob et al., Ann. Inst. Pasteur (Paris) 84, 222-224, 1953).

Les bactériocines suscitent un intérêt grandissant dans le monde de la recherche et de l'industrie ; elles pourraient fournir des solutions alternatives à l'utilisation d'antibiotiques, notamment dans l'élevage (Luchansky, Antonie Van Leeuwenhoek 76, 335, 1999 ; O'Sullivan et al., Biochimie 84, 593-604, 2002).

De nombreux systèmes d'expression hétérologues de ces bactériocines se sont développés depuis quelques années. Notamment, Morisset et al. (Morisset et al., Appl. Environ. Microbiol., 70, 4672-4680, 2004) ont exprimé des variants de la mésentricine Y105, bactériocine de classe IIa produite par *Leuconostoc mesenteroides subsp. mesenteroides Y105,* chez *Leuconostoc mesenteroides subsp. dextranicum* DSM20484. De même, Flynn et al. (Microbiol., 148, 973-984, 2002) ont réalisé l'expression de ABP-118, bactériocine de classe IIb produite à l'origine par *Lactobacillus salivarius subsp. salivarius* UCC118, chez les hôtes *Lactobacillus plantarum, Lactococcus lactis* et *Bacillus cereus.*

En outre, plusieurs essais ont été menés pour exprimer des bactériocines chez la bactérie *Escherichia coli* (McCormick et al., Appl. Environ. Microbiol., 64, 4757-4766, 1998; Garneau et al., Appl. Environ. Microbiol., 69, 1352-1358, 2003; Biet et al., Microbiol., 144, 2845-2854, 1998 ; Miller et al., Appl. Environ. Microbiol., 64, 14-20, 1998; Richard et al., J. Bacteriol., 186, 4276-4284, 2004; Kloche et al., Appl. Microbiol. Biotechnol., 67:532-538, 2005), la levure *Saccharomyces cerevisiae* (Schoeman et al., Yeast, 15, 647-656, 1999; Van Reenen et al., Int. J. Food Microbiol., 81, 29-40, 2003) et chez des bactéries lactiques (Rodriguez et al., Int. J. Food Microbiol., 80, 101-116, 2003).

De nombreux travaux sont donc menés en vue de l'identification de nouvelles bactériocines et en vue de la production de ces bactériocines.

L'écosystème digestif est constitué par une microflore abondante et très complexe regroupant des bactéries, des levures et des *Archeae.* Ce microbiote est essentiellement anaérobie et l'on retrouve principalement des bactéries des genres *Bacteroides, Eubacterium, Clostridium, Ruminococcus, Bifidobacterium* et *Fusobacterium* (Suau et al., Appl. Environ. Microbiol. 65, 4799-4807, 1999). La microflore a un impact important sur la santé de l'hôte. Elle est notamment impliquée dans la toxification et la détoxication de composés métaboliques issus de l'alimentation (Hughes and Rowland Microbial Ecology Health Disease 2, 179-185, 2000). Elle est également capable de moduler l'expression de fonctions entérocytaires (Bry et al., Science 273,1380-1383, 1996 ; Hooper et al., Science 291, 881-884, 2001). Enfin, elle joue un rôle primordial dans la protection de l'hôte contre l'envahissement par des bactéries exogènes potentiellement pathogènes (Ducluzeau et al., Microbial Ecology and Intestinal Infections, 1988 ; Fons et al., Microbial Ecology in Health and Disease 2, 240-246, 2000).

Parmi les pathogènes intestinaux connus, on trouve *Clostridium perfringens,* bactérie à Gram positif, anaérobie stricte, capable de sporuler et très répandue dans l'environnement. Ce pathogène peut provenir de l'alimentation, mais peut aussi être présent à faible concentration dans l'intestin et se mettre à proliférer et sécréter des toxines sous l'effet d'un stress. Les souches de *Clostridium perfringens* sont classées en 5 toxinotypes en fonction des toxines qu'elles produisent (Petit et al., Trends Microbiol. 7, 104-110, 1999). Les souches de C. *perfringens* de type A sont responsables de maladies gastro-intestinales chez l'Homme. En 1997, plus de 245000 cas d'infections à *C. perfringens* ont été recensés aux Etats-Unis. Ceci a conduit à l'hospitalisation de 41 personnes dont 7 n'ont pas survécu (Mead et al., Emerg. Infect. Dis. 5, 607-625, 1999). Les souches de C. *perfringens* de type A et C peuvent être respectivement à l'origine d'entérite nécrotique chez les volailles et les porcs. Chez les volailles, l'entérite nécrotique est une pathologie aiguë, d'évolution rapide dont la mortalité peut atteindre 1 à 2% par jour. En plus de son incidence sur le bien-être des animaux, cette pathologie peut donc avoir une influence économique non négligeable. Jusqu'en 1999, cette maladie était bien contrôlée par l'usage d'antibiotiques comme facteurs de croissance. Mais, l'Union européenne a interdit leur emploi dans l'alimentation animale par crainte de sélectionner les bactéries résistantes et donc de voir diminuer l'efficacité des antibiotiques chez l'Homme. Depuis cette interdiction, l'entérite nécrotique provoquée par *Clostridium perfringens* chez la volaille et le porc, n'est plus contrôlée en Europe. Le nombre de cas déclarés au Réseau National d'Observations Epidémiologiques en Aviculture (RNOEA) (AFSSA Ploufragan) a considérablement augmenté en 1999 et 2000 (Valancony, Bulletin des GTV 12, 9-12, 2001).

A ce jour, la recherche de solutions alternatives pour contrôler et traiter cette maladie est donc de première importance.

*Ruminococcus gnavus* est une bactérie anaérobie stricte appartenant à la famille des *Lachnospiraceae,* dans l'ordre des *Clostridiales.* Dabard et al. (Appl. Environ. Microbiol., 67, 4111-4118, 2001) ont montré que la souche *Ruminococcus gnavus* E1, isolée à partir de la flore dominante de l'Homme, est capable de produire une substance antimicrobienne, appelée ruminococcine A ou RumA, qui s'accumule dans le surnageant de culture. Il s'agit d'une bactériocine appartenant à la famille des lantibiotiques, active contre différentes souches de *Clostridium sp.* pathogènes. Gomez et al. (J. Bacteriol., 184, 18-28, 2002) ont montré que l'expression des gènes impliqués dans la biosynthèse de la ruminococcine A est induite en présence de trypsine. Les mêmes auteurs montrent également que certaines enzymes digestives pourraient inhiber l'induction de la production de RumA.

Les inventeurs se sont donc intéressés à d'autres bactériocines.

La présente invention concerne les peptides RumC1, RumC2 et RumC3 présentant une activité antibactérienne contre *Clostridium perfringens,* ainsi que les gènes codant pour ces peptides.

## Description des séquences

SEQ ID No. 1 : Séquence peptidique conservée des peptides RumC de *Ruminococcus gnavus* E1
SEQ ID No. 3 : Séquence peptidique des peptides RumC de *Ruminococcus gnavus* E1 déterminée expérimentalement par la méthode de dégradation d'Edman
SEQ ID No. 4 : Séquence peptidique du peptide RumC1 de *Ruminococcus gnavus* E1 déduite de SEQ ID No. 7
SEQ ID No. 5 : Séquence peptidique du peptide RumC2 de *Ruminococcus gnavus* E1 déduite de SEQ ID No. 8
SEQ ID No. 6 : Séquence peptidique du peptide RumC3 de *Ruminococcus gnavus* E1 déduite de SEQ ID No. 9
SEQ ID No. 7 : Séquence nucléotidique du gène *rumC1* de *Ruminococcus gnavus* E1
SEQ ID No. 8 : Séquence nucléotidique du gène *rumC2* de *Ruminococcus gnavus* E1
SEQ ID No. 9 : Séquence nucléotidique du gène *rumC3* de *Ruminococcus gnavus* E1
SEQ ID No. 10, 11 et 12 : Séquences peptidiques déterminées expérimentalement

## Description de l'invention

La présente invention concerne un peptide ayant une activité antimicrobienne et plus spécifiquement contre les souches de Clostridium perfringens et caractérisé en ce qu'il comprend un peptide choisi parmi les peptides suivants : le peptide de la séquence SEQ ID No. 1, un peptide comprenant un peptide présentant au moins 80 % d'identité avec le polypeptide de la SEQ ID No. 1, le peptide de la séquence SEQ ID No. 2, et un peptide comprenant un peptide présentant au moins 80 % d'identité avec le polypeptide de la SEQ ID No. 2. Selon un mode de réalisation de la présente invention, ce peptide comprend en outre le peptide de la séquence SEQ ID No. 3. Selon un autre mode de réalisation de la présente invention, ce peptide comprend un peptide choisi parmi les peptides de séquences SEQ ID No. 4, 5 ou 6.

La présente invention concerne également un polynucléotide codant pour une activité antimicrobienne et plus spécifiquement contre les souches de Clostridium perfringens caractérisé en ce qu'il est comprend un polynucléotide choisi parmi le polynucléotide selon l'une quelconque des séquences SEQ ID No. 7, 8 ou 9, un polynucléotide qui s'hybride dans des conditions de moyenne à forte stringence au polynucléotide selon l'une quelconque des séquences SEQ ID No. 7, 8 ou 9 et un polynucléotide codant pour un polypeptide tel que ci-dessus défini.

La présente invention concerne également une cassette d'expression caractérisée en ce qu'elle comprend dans le sens de la transcription un promoteur fonctionnel dans un organisme hôte, un polynucléotide tel que ci-dessus défini et une séquence terminatrice dans le même organisme hôte.

La présente invention concerne encore un vecteur comprenant un polynucléotide tel que ci-dessus défini et/ou une cassette d'expression telle que ci-dessus définie.

La présente invention concerne aussi un organisme hôte transformé avec un polynucléotide tel que ci-dessus défini, une cassette d'expression telle que ci-dessus définie et/ou un vecteur tel que ci-dessus défini.

La présente invention concerne une souche de *Ruminococcus gnavus* déposée à la CNCM (Collection Nationale de Cultures de Microorganismes, Institut Pasteur, 25 rue du Docteur Roux, F-75015 Paris) le 19 décembre 2006 sous le numéro CNCM I-3705, ainsi qu'une souche bactérienne à l'état isolée non modifiée génétiquement produisant un peptide tel que défini ci-dessus.

La présente invention concerne un mélange protéique ou moût de fermentation susceptible d'être obtenu par un organisme hôte ou par la souche ci-dessus définie et plus particulièrementt par un procédé de préparation comprenant les étapes de mise en culture de la souche citée ci-dessus ou d'un organisme hôte transformé avec un polynucléotide, une cassette d'expression et/ou un vecteur selon l'invention dans des conditions d'induction de l'expression du peptide, et la récupération du surnageant de culture comprenant le peptide,-optionnellement, casse des cellules et purification de l'extrait cellulaire.

La présente invention concerne également une composition comprenant un peptide tel que ci-dessus défini, un organisme hôte tel que ci-dessus défini, une souche telle que ci-dessus définie, un moût de fermentation d'un organisme hôte tel que ci-dessus défini ou un moût de fermentation d'une souche telle que ci-dessus définie. Selon un mode de réalisation de la présente invention, la composition se présente sous forme liquide ou sous forme de poudre.

La présente invention concerne également un additif nutritionnel comprenant un peptide tel que ci-dessus défini, un organisme hôte tel que ci-dessus défini, une souche telle que ci-dessus définie, un moût de fermentation d'un organisme hôte tel que ci-dessus défini ou un moût de fermentation tel

La présente invention concerne également un additif nutritionnel comprenant un peptide tel que ci-dessus défini, un organisme hôte tel que ci-dessus défini, une souche telle que ci-dessus définie, un moût de fermentation d'un organisme hôte tel que ci-dessus défini ou un moût de fermentation tel que ci-dessus défini. Selon un mode de réalisation de la présente invention, l'additif se présente sous forme liquide ou sous forme de poudre.

La présente invention concerne aussi un aliment pour animaux caractérisé en ce qu'il comprend une base nutritionnelle pour animaux et un additif nutritionnel tel que ci-dessus défini.

La présente invention concerne également l'utilisation d'un peptide tel que défini ci-dessus, d'un organisme hôte tel que défini ci-dessus, d'une souche telle que définie ci-dessus, d'un moût de fermentation d'un organisme hôte tel que défini ci-dessus ou d'un moût de fermentation d'une souche telle que définie ci-dessus pour la fabrication d'un médicament, d'un additif nutritionnel ou d'un aliment pour animaux. Selon un mode de réalisation de la présente invention, ce médicament ou cet additif nutritionnel est destiné à prévenir ou traiter l'entérite nécrotique chez les volailles ou chez les porcs. Selon un autre mode de réalisation de la présente invention, ce médicament ou cet additif nutritionnel est destiné à prévenir ou traiter les maladies gastro-intestinales chez l'Homme.

Enfin, la présente invention concerne également l'utilisation non thérapeutique d'un peptide tel que défini ci-dessus pour traiter les animaux. Selon un mode de réalisation de la présente invention, le peptide provient d'une souche endogène ou d'une souche exogène de l'animal. Selon un autre mode de réalisation de la présente invention, le peptide provient d'une souche endogène de l'animal et la production du peptide par ladite souche endogène est favorisée. Selon un autre mode de réalisation encore de la présente invention, le peptide provient d'une souche endogène de l'animal et la croissance de ladite souche endogène est favorisée.

Peptides

La présente invention concerne donc des peptides ayant une activité antimicrobienne. De préférence, ces peptides sont isolés de

75015 Paris) le 19 décembre 2006 sous le numéro CNCM I-3705, c'est-à-dire la souche LEM 9-17.

On entend par "activité antimicrobienne" la capacité à inhiber la croissance ou le développement de bactéries cibles ou la capacité à tuer des bactéries cibles en particulier, l'activité microbienne selon la présente invention est dirigée contre les souches de Clostridium perfringens. Les techniques de mesure de l'activité antimicrobienne sont connues de l'homme du métier. L'activité antimicrobienne est mise en évidence dans la présente invention par un test d'activité sur la souche *Clostridium perfringens* CpA mise en culture sur milieu gélosé. L'échantillon contenant l'un des peptides de l'invention est déposé dans des puits formés dans le milieu gélosé. L'activité antimicrobienne est mise en évidence lorsqu'est formé un halo d'inhibition autour du puits.

Les séquences peptidiques des peptides RumC1, RumC2 et RumC3 de *Ruminococcus gnavus* E1 sont respectivement représentées par les séquences SEQ ID No. 4, SEQ ID No. 5 et SEQ ID No. 6. Ces séquences sont respectivement déduites des séquences nucléotidiques des gènes *rumC1, rumC2* et *rumC3* représentées respectivement par les séquences SEQ ID No. 7, SEQ ID No. 8 et SEQ ID No. 9.

L'invention se rapporte également à des fragments de ces peptides RumC1, RumC2 et RumC3 de *Ruminococcus gnavus* E1, ayant une activité antimicrobienne. Le terme "fragment" d'un peptide désigne un peptide comprenant une partie mais pas la totalité du peptide dont il est dérivé. L'invention concerne ainsi les peptides des séquences SEQ ID No. 1, SEQ ID No. 2 et SEQ ID No. 3.

Ces fragments conservent leur activité antimicrobienne. L'invention concerne donc les fragments biologiquement actifs. Le terme "fragment biologiquement actif" désigne un fragment d'un polypeptide conservant la fonction du polypeptide dont il est dérivé.

L'invention se rapporte également à des peptides ayant une activité antimicrobienne et présentant au moins 80%, 90%, 95%, 98% et préférentiellement au moins 99% d'acides aminés identiques avec l'un quelconque des peptides des séquences SEQ ID No. 1, 2 ou 3.

L'invention se rapporte également à des peptides ayant une activité antimicrobienne et présentant au moins 80%, 90%, 95%, 98% et préférentiellement au moins 99% d'acides aminés identiques avec l'un quelconque des peptides des séquences SEQ ID No. 1, 2 ou 3.

Les méthodes de préparation des peptides de séquences SEQ ID No. 1, SEQ ID No. 2 et SEQ ID No. 3 sont connues de l'homme du métier.

Les peptides RumC sont secrétés (ou relargués) par les bactéries dans l'environnement extracellulaire. Il est possible que l'un quelconque des peptides de séquences SEQ ID No. 4, 5 et/ou 6 comprenne un peptide signal d'un nombre déterminé d'acides aminés. Dans ce cas, l'invention concerne également le peptide mature obtenu après clivage du peptide signal. Selon un mode de réalisation de la présente invention, l'invention se rapporte aux peptides dont les séquences sont comprises entre la position 20 et la position 63 des séquences SEQ ID No. 4, 5 et 6.

Dans un autre mode de réalisation, le peptide signal potentiel du peptide SEQ ID No. 4, 5 ou 6 peut être remplacé par un peptide signal hétérologue pour réaliser l'expression et la secrétion de ce peptide par un organisme hôte hétérologue.

L'invention a aussi pour objet un peptide ayant une activité antimicrobienne et présentant au moins 80 % d'identité avec le peptide de la SEQ ID No. 4, 5 ou 6. Selon un mode de réalisation de la présente invention, ce peptide est isolé à partir d'une souche de *Ruminococcus gnavus.* Selon un autre mode de réalisation de la présente invention, ce peptide est isolé à partir d'autres souches de *Ruminococcus* ou d'autres bactéries. Alternativement, ce peptide peut être obtenu par synthèse chimique.

L'invention a pour objet un peptide présentant au moins 90%, 95%, 98% et préférentiellement au moins 99% d'acides aminés identiques avec l'un quelconque des peptides des séquences SEQ ID No. 4, 5 ou 6.

Par "acides aminés identiques", on entend des acides aminés invariants ou inchangés entre deux séquences. Ces peptides peuvent présenter une délétion, une addition ou une substitution d'au moins un acide aminé par rapport aux peptides représentés par les séquences SEQ ID No. 4, 5 ou 6. Sont considérés comme acides aminés inchangés entre deux séquences des acides aminés modifiés après transcription par exemple déshydratation, formation de ponts monosulfures, lanthionines...

L'invention a également pour objet des peptides présentant au moins 80%, 90%, 95%, 98% et préférentiellement au moins 99% de similarité avec l'un quelconque des peptides des séquences SEQ ID No. 4, 5 ou 6.

Par "similarité", on entend la mesure de la ressemblance entre séquences protéiques ou nucléiques. Ces peptides peuvent présenter une délétion, une addition ou une substitution d'au moins un acide aminé par rapport aux peptides représentés par les séquences SEQ ID No. 4, 5 ou 6. Le degré de similarité entre deux séquences, quantifié par un score, est basé sur le pourcentage d'identités et/ou de substitutions conservatrices des séquences.

Les méthodes de mesure et d'identification du degré d'identité et du degré de similarité entre polypeptides sont connues de l'homme du métier. L'alignement des séquences est par exemple réalisé au moyen de Vector NTi 9.1.0, programme d'alignement AlignX (Clustal W algorithm) (Invitrogen INFORMAX, http://www.invitrogen.com) ou en utilisant l'outil CLUSTAW (http://www.ebi.ac.uk/clustalw/).

Les peptides selon l'invention peuvent être isolés ou purifiés de leur environnement naturel. Les peptides peuvent être préparés au moyen de différents procédés. Ces procédés sont notamment la purification à partir de sources naturelles telles que des bactéries exprimant naturellement ces peptides, la production de peptides recombinants par des cellules hôtes appropriées et leur purification ultérieure, la production par synthèse chimique ou, enfin, une combinaison de ces différentes approches. Ainsi, les peptides des séquences SEQ ID No. 1 à 6 de la présente invention peuvent être isolés à partir de la souche de *Ruminococcus gnavus* déposée à la CNCM le 19 décembre 2006 sous le numéro CNCM I-3705. Dans un autre mode de réalisation, les peptides de la présente invention sont isolés à partir d'organisme hôtes recombinants exprimant un peptide RumC selon l'invention ou un fragment du peptide RumC présentant une activité antimicrobienne.

L'invention a également pour objet des protéines de fusion, des protéines recombinantes ou des protéines chimères comprenant les peptides selon l'invention.

Les peptides de l'invention peuvent être isolés à partir de contenu caecal de rat monoxénique hébergeant la souche *Ruminococus gnavus* E1 et à partir d'une souche de *Ruminococcus gnavus* mutante, plus précisément la souche de *Ruminococcus gnavus* déposée à la CNCM le 19 décembre 2006 sous le numéro CNCM I-3705. Ces peptides présentent une activité antimicrobienne, mise en évidence par un test d'activité sur *Clostridium perfringens.*

La spectrométrie de masse a permis de déterminer la masse moléculaire approximative du peptide RumC selon l'invention. La masse moléculaire se situe entre 4000 et 4600 Da, plus précisément entre 4100 et 4500 Da.

Selon un mode de réalisation de la présente invention, le peptide est adapté à une utilisation en nutrition ou en pharmacie, par exemple une utilisation en nutrition animale.

On entend par "peptide adapté à l'utilisation dans la nutrition ou la pharmacie", un peptide dont les caractéristiques sont telles qu'il convient pour la nutrition ou la pharmacie. Les caractéristiques essentielles à une utilisation en nutrition ou en pharmacie sont notamment le pH auquel le peptide peut résister. En effet, le pH du système digestif des animaux et de l'Homme est acide et il est donc essentiel que le peptide soit résistant à ce pH. Une autre caractéristique essentielle à une utilisation en nutrition est la température à laquelle la substance antimicrobienne est active. En effet, la mise en forme de substance antimicrobienne dans un médicament, un additif nutritionnel ou un aliment pour animaux, par exemple, implique des traitements et une température supérieure à la température ambiante. L'activité des antimicrobiens utilisés doit donc être stable dans les conditions des procédés, notamment les conditions de températures.

Selon un mode de réalisation de la présente invention, le peptide, ou un mélange de peptides selon l'invention, présente une activité antimicrobienne à pH neutre et conserve son activité antimicrobienne à un pH acide, par exemple inférieur à 7, de préférence inférieur à 4,4.

Selon un mode de réalisation de la présente invention, le peptide, ou un mélange de peptides selon l'invention, présente une activité antimicrobienne à 37°C et conserve cette activité à des températures inférieures et supérieures à la température ambiante, par exemple supérieure à 50°C.

L'invention concerne également un peptide présentant une activité antimicrobienne, isolé à partir de contenu caecal de rat monoxénique ou à partir d'une souche *Ruminococcus gnavus* mutante, présentant une activité contre les souches *Clostridium perfringens,* ayant une masse moléculaire comprise entre 4000 et 4600 Da telle que déterminée par spectrométrie de masse et étant résistante à un pH inférieur ou égal à 7. Selon un mode de réalisation, le peptide comprend la séquence SEQ ID No.1 et/ou la séquence SEQ ID No.2. Selon un autre mode de réalisation, il comprend en outre le peptide de la séquence SEQ ID No. 3. Avantageusement, le peptide comprend un peptide choisi parmi l'un quelconque des peptides de séquence SEQ ID No. 4, 5 ou 6.

L'invention concerne également un peptide présentant une activité antimicrobienne, susceptible d'être obtenu à partir d'une souche *Ruminococcus gnavus* mutante, par exemple la souche de *Ruminococcus gnavus* déposée à la CNCM le 19 décembre 2006 sous le numéro CNCM I-3705. Selon un mode de réalisation, le peptide présente une activité antimicrobienne à l'égard des souches de *Clostridium perfringens.* Selon un autre mode de réalisation, il présente une masse moléculaire comprise entre 4000 et 4600 Da telle que déterminée par spectrométrie de masse, préférentiellement comprise entre 4100 et 4500 Da. Selon un autre mode de réalisation, le peptide présente une activité antimicrobienne à pH neutre et conserve son activité antimicrobienne à un pH acide, par exemple inférieur à 7, de préférence inférieur à 4,4. Selon un mode de réalisation, le peptide comprend la séquence SEQ ID No.1 et/ou la séquence SEQ ID No.2. Selon un autre mode de réalisation, il comprend en outre le peptide de la séquence SEQ ID No. 3. Avantageusement, le peptide comprend un peptide choisi parmi l'un quelconque des peptides de séquence SEQ ID No. 4, 5 ou 6.

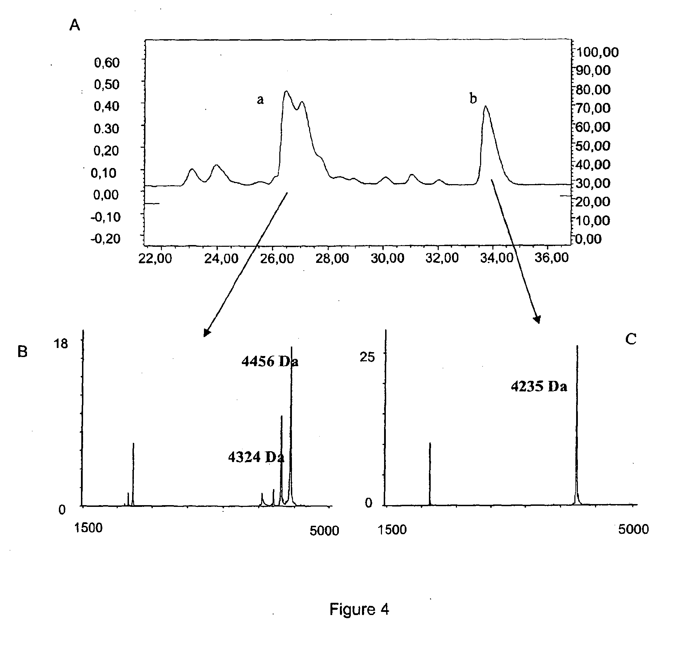

L'invention concerne également un peptide présentant un potentiel antimicrobien isolé à partir de contenu caecal de rat monoxénique, correspondant au chromatogramme de la figure 4A, obtenu à 214nm par HPLC en phase inverse, présentant une activité contre différentes souches de *Clostridium perfringens,* en particulier de toxinotype A.

Polynucléotides

L'invention concerne aussi des polynucléotides codant pour une activité antimicrobienne. De préférence, ces polynucléotides codent pour une activité antimicrobienne de *Ruminococcus* et plus préférentiellement encore contre les souches de Clostridium perfringens.

Selon la présente invention, on entend par "polynucléotide" une chaîne nucléotidique simple brin ou son complémentaire pouvant être de type ADN ou ARN, ou une chaîne nucléotidique double brin pouvant être de type ADN complémentaire ou génomique. De préférence, les polynucléotides de l'invention sont de type ADN, notamment ADN double brin. Le terme "polynucléotide" désigne également les polynucléotides modifiés.

Les polynucléotides de la présente invention peuvent être isolés ou purifiés de leur environnement naturel. Les polynucléotides de la présente invention peuvent également être préparés par synthèse chimique ou par des techniques classiques de biologie moléculaire telles que décrites par Sambrook, Fristsch et Maniatis, dans leur ouvrage intitulé "Molecular cloning: a laboratory manual", édition : Cold Spring Harbor Laboratory Press, Cold Spring Harbor, NY, 1989.

L'invention se rapporte au polynucléotide selon l'une quelconque des séquences SEQ ID No. 7, 8 ou 9. L'invention se rapporte également au polynucléotide qui s'hybride au polynucléotide selon l'une quelconque des séquences SEQ ID No. 7, 8 ou 9. L'invention se rapporte également au polynucléotide codant pour un peptide présentant une activité antimicrobinenne tel que ci-dessus défini.

L'invention concerne aussi un polynucléotide présentant au moins 80%, 85%, 90%, 95%, 98% et de préférence au moins 99% d'identité avec le polynucléotide selon l'une quelconque des séquences SEQ ID No. 7, 8 ou 9. Ce polynucléotide code pour une activité antimicrobienne. Selon un mode de réalisation, le polynucléotide code pour une activité antimicrobienne à l'égard des souches *Clostridium perfringens.*

Par "nucléotides identiques", on entend des nucléotides invariants ou inchangés entre deux séquences. Ce polynucléotide peut présenter une délétion, une addition ou une substitution d'au moins un nucléotide par rapport au polynucléotide de référence.

L'invention concerne aussi un polynucléotide présentant au moins 80%, 85%, 90%, 95%, 98% et de préférence au moins 99% de similarité avec le polynucléotide selon l'une quelconque des séquences SEQ

ID No. 7, 8 ou 9. Ce polynucléotide code pour une activité antimicrobienne. Selon un mode de réalisation, le polynucléotide code pour une activité antimicrobienne à l'égard des souches *Clostridium perfringens.*

Par "similarité", on entend la mesure de la ressemblance entre séquences protéiques ou séquences nucléiques. Ce polynucléotide peut présenter une délétion, une addition ou une substitution d'au moins un nucléotide par rapport au polynucléotide de référence. Le degré de similarité entre deux séquences, quantifié par un score, est basé sur le pourcentage d'identités et/ou de substitution conservatrices des séquences.

Les méthodes de mesure et d'identification du degré d'identité et du degré de similarité entre les séquences d'acides nucléiques sont connues de l'homme du métier. L'alignement des séquences est par exemple réalisé au moyen de Vector NTi 9.1.0, programme d'alignement AlignX (Clustal W algorithm) (Invitrogen INFORMAX, http://www.invitrogen.com) ou en utilisant l'outil CLUSTAW (http://www.ebi.ac.uk/clustalw/).

L'invention concerne aussi des polynucléotides capables dé s'hybrider de manière sélective avec le polynucléotide selon l'une quelconque des séquences SEQ ID No. 7, 8 ou 9. De préférence, dans le cadre de l'invention, l'hybridation sélective est effectuée dans des conditions de moyenne stringence et préférentiellement dans des conditions de forte stringence. Par "séquence capable de s'hybrider de manière sélective", on entend selon l'invention les séquences qui s'hybrident avec la séquence de référence à un niveau supérieur au bruit de fond de manière significative. Le niveau du signal généré par l'interaction entre la séquence capable de s'hybrider de manière sélective et les séquences de référence est généralement 10 fois, de préférence 100 fois plus intense que celui de l'interaction des autres séquences d'ADN générant le bruit de fond. Les conditions d'hybridation stringentes permettant une hybridation sélective sont connues de l'homme du métier. En général, la température d'hybridation et de lavage est inférieure d'au moins 5°C au Tm de la séquence de référence à un pH donné et pour une force ionique donnée. Typiquement la température d'hybridation est d'au moins 30°C pour un polynucléotide de 15 à 50 nucléotides et d'au moins 60°C pour un polynucléotide de plus de 50 nucléotides. A titre d'exemple, l'hybridation est réalisée dans le tampon suivant : 6X SSC, 50 mM Tris-HCl (pH 7.5), 1 2X SSC, 0,1% SDS, à moyenne stringence dans un tampon 0,5X SSC, 01% SDS et à forte stringence dans un tampon 0,1X SSC, 0,1%SDS. L'hybridation peut bien entendu être effectuée selon d'autres méthodes usuelles connues de l'homme du métier (voir notamment Sambrook, Fristsch et Maniatis, dans leur ouvrage intitulé "Molecular cloning: a laboratory manual", édition : Cold Spring Harbor Laboratory Press, Cold Spring Harbor, NY, 1989). De préférence, les polynucléotides s'hybridant de manière sélective à un polynucléotide de référence conservent la fonction de la séquence de référence. Dans le cas présent, les polynucléotides s'hybridant de manière sélective avec le polynucléotide selon l'une quelconque des séquences SEQ ID No. 7, 8 ou 9. codent pour une activité anti-microbienne.

L'invention se rapporte de manière générale aux polynucléotides codant pour les peptides selon l'invention. En raison de la dégénerescence du code génétique, différents polynucléotides peuvent coder pour un même polypeptide.

Cassettes d'expression

Selon un mode de réalisation de l'invention, un polynucléotide codant pour un peptide selon l'invention est inséré dans une cassette d'expression en utilisant des techniques de clonage bien connues de l'homme du métier. Cette cassette d'expression comprend les éléments nécessaires à la transcription et à la traduction des séquences codant pour les polypeptides selon l'invention.

Avantageusement, cette cassette d'expression comprend à la fois des éléments permettant de faire produire un peptide par une cellule hôte et des éléments nécessaires à la régulation de cette expression.

Ces cassettes d'expression comprennent dans le sens de la transcription:

- un promoteur fonctionnel dans un organisme hôte ;
- un polynucléotide selon l'invention ;
- une séquence terminatrice fonctionnelle dans le même organisme hôte.

Tout type de séquence promotrice peut être utilisée dans les cassettes d'expression selon l'invention. Le choix du promoteur dépendra notamment de l'organisme hôte choisi pour l'expression du gène d'intérêt. Certains promoteurs permettent une expression constitutive alors que d'autres promoteurs sont au contraire inductibles.

Parmi les promoteurs fonctionnels dans les champignons, on citera notamment celui de glyceraldehyde-3-phosphate deshydrogenase *d'Aspergillus nidulans* (Roberts et al., Current Genet. 15, 177-180, 1989).

Parmi les promoteurs fonctionnels dans les bactéries, on citera notamment celui de la RNA polymérase du bacteriophage T7 (Studier et al., Methods in enzymology, 185, 60-89, 1990).

Parmi les promoteurs fonctionnels dans les levures, on citera le promoteur du gène *GAL1* (Elledge et al., Proc Natl Acad Sciences, USA. 88, 1731-1735, 1991) ou les promoteurs *GAL4* et *ADH* de *S.cerevisiae.* Tous ces promoteurs sont décrits dans la littérature et bien connus de l'homme du métier.

Pour l'expression dans *Penicillium funiculosum,* on choisira par exemple des cassettes d'expression comprenant un promoteur histone H4B, un promoteur acide aspartyl protéase ou un promoteur csl13 (WO 00/68401 ).

Pour l'expresssion dans la levure *Pichia pastoris,* on choisira par exemple des cassettes d'expression comprenant le promoteur AOX1 inductible par le méthanol (Tschopp et al., Biotechnology, 5, 1305-1308, 1987) ou le promoteur constitutif fort GAP (Waterham et al., Gene 186, 37-44, 1997).

Pour l'expression dans *Schizosacchromyces pombe,* on choisira par exemple des cassettes d'expression comprenant le promoteur de régulation *Nmt1* reprimé par la thiamine et actictivé en abscence de thiamine (Maundrell, J. Biol. Chem. 265, 10857-10864, 1989)

Les cassettes d'expression selon la présente invention peuvent en outre inclure toute autre séquence nécessaire à l'expression des polypeptides ou des polynucléotides comme par exemple des éléments de régulation ou des séquences signal permettant la sécrétion des polypeptides produits par l'organisme hôte. On peut notamment utiliser toute séquence de régulation permettant d'augmenter le niveau d'expression de la séquence codante insérée dans la cassette d'expression. Selon l'invention, on peut notamment utiliser, en association avec la séquence de régulation promotrice, d'autres séquences de régulation, qui sont situées entre le promoteur et la séquence codante, telles que des activateurs de transcription ("enhancer").

En outre, les cassettes d'expression selon la présente invention peuvent inclure toute autre séquence nécessaire à la sécrétion des polypeptides produits par l'organisme hôte telles que des séquences signal. Pour la sécrétion par *Pichia pastoris,* on peut par exemple utiliser la séquence du facteur α comme signal de sécrétion.

Une grande variété de séquences terminatrices sont utilisables dans les cassettes d'expression selon l'invention, ces séquences permettent la terminaison de la transcription et la polyadénylation de l'ARNm. Toute séquence terminatrice fonctionnelle dans l'organisme hôte sélectionné peut être utilisée.

Pour l'expression dans *Penicillium funiculosum,* on choisira par exemple des cassettes d'expression comprenant un terminateur histone H4.B, un terminateur acide aspartyl protease ou un terminateur csl13 (WO 00/68401).

La présente invention a également pour objet un polynucléotide comprenant une cassette d'expression selon l'invention, avantageusement les cassettes d'expression selon la présente invention sont insérées dans un vecteur.

Vecteurs

La présente invention concerne également des vecteurs de clonage ou d'expression pour la transformation d'un organisme hôte comprenant au moins un polynucléotide ou une cassette d'expression selon la présente invention. Ce vecteur peut notamment correspondre à un plasmide, un cosmide, un bactériophage ou un virus dans lequel est inséré un polynucléotide ou une cassette d'expression selon l'invention. Les techniques de construction de ces vecteurs et d'insertion d'un polynucléotide de l'invention dans ces vecteurs sont connues de l'homme du métier. De manière générale, tout vecteur capable de se maintenir, de s'autorépliquer ou de se propager dans une cellule hôte afin d'induire notamment l'expression d'un polynucléotide ou d'un peptide peut être utilisé. L'homme du métier choisira les vecteurs appropriés en fonction de l'organisme hôte à transformer, et en fonction de la technique de transformation mise en oeuvre.

Les vecteurs de la présente invention sont notamment utilisés pour transformer un organisme hôte en vue de la réplication du vecteur et/ou de l'expression d'un peptide selon l'invention dans l'organisme hôte.

L'invention concerne aussi une méthode pour préparer un peptide selon l'invention comprenant les étapes suivantes:

- on transforme un organisme hôte avec un vecteur d'expression comprenant une cassette d'expression selon l'invention et/ou avec un polynucléotide selon l'invention,
- on isole les peptides produits par l'organisme hôte.

Organismes hôtes

La présente invention a également pour objet, un procédé de transformation d'un organisme hôte par intégration dans ledit organisme hôte d'au moins un polynucléotide, d'au moins une cassette d'expression ou d'au moins un vecteur selon l'invention. Le polynucléotide peut être intégré dans le génome de l'organisme hôte ou se repliquer de manière stable dans l'organisme hôte. Les méthodes de transformation des organismes hôtes sont connus de l'homme du métier et largement décrites dans la littérature.

La présente invention concerne également un organisme hôte transformé avec un polynucléotide, une cassette d'expression ou un vecteur selon l'invention.

Par "organisme hôte", on entend en particulier selon l'invention tout organisme mono ou pluricellulaire, inférieur ou supérieur, en particulier choisi parmi les bactéries, les levures et les champignons. En particulier, par "organisme hôte", on entend un organisme non humain. De manière avantageuse, les levures sont choisies parmi par exemple *Pichia pastoris, Saccharomyces cerevisae, Yarrowia lipolytica* et *Schwanniomyces occidentalis.* Les champignons sont par exemple choisis parmi les *Aspergillus,* les *Trichoderma* et les *Penicilliums,* préférentiellement parmi *Penicillium funiculosum, Trichoderma reesei, Aspergillus niger, Aspergillus awamori, Aspergillus kawachii* et *Trichoderma koningii.* Dans un mode de réalisation de l'invention, l'organisme hôte est une souche de *Penicillium funiculosum* dans laquelle on exprime ou sur-exprime un peptide selon l'invention. Dans un autre mode de réalisation, l'organisme hôte est une souche de *Debaryomyces castellii* dans laquelle on exprime ou sur-exprime un peptide selon l'invention. Dans un autre mode encore de réalisation, l'organisme hôte est une souche de *Ruminococcus gnavus* dans laquelle on exprime ou sur-exprime un peptide selon l'invention.

Les techniques de construction de vecteurs, de transformation d'organismes hôtes et d'expression de protéines hétérologues dans ces organismes sont largement décrites dans la littérature notamment par Sambrook, Fristsch et Maniatis, dans l'ouvrage intitulé "Molecular cloning: a laboratory manual", édition : Cold Spring Harbor Laboratory Press, Cold Spring Harbor, NY, 1989 ou par Ausubel et al., dans l'ouvrage intitulé "Current Protocols in Molecular Biology", édition : Greene Publishing Associates, Inc., and John Wiley and Sons, NY, 1992.

Souches

La nouvelle souche de *Ruminococcus gnavus* a été déposée à la CNCM (Collection Nationale de Cultures de Microorganismes, Institut Pasteur, 25 rue du Docteur Roux, F-75015 Paris) le 19 décembre 2006 sous le numéro CNCM I-3705. La CNCM est une autorité de dépôt internationale selon l'Article 7 du Traité de Budapest.

La nouvelle souche a été obtenue par mutagenèse aléatoire de la souche R. *gnavus* L14. Cette souche est un mutant spontané de *R. gnavus* E1 ayant perdu la capacité de produire RumA *in vitro* mais synthétisant toujours RumC *in vivo.* Un agent alkylant puissant, le N-methyl-N'-nitro-N-nitrosoguanidine (NG) a été utilisé pour la mutagènese. Aucune modification génétique n'a été obtenue par les techniques de recombinaison utilisant un ADN ou un ARN d'une autre origine.

Le milieu de culture de la souche LEM 9-17 est de préférence est milieu BHI supplémenté en extrait de levure et en hémine (BHI-YH).

Mélange protéique, moût de fermentation

L'invention concerne aussi un procédé de préparation d'un peptide présentant une activité antimicrobienne selon l'invention, ledit procédé comprenant les étapes suivantes :

a) mise en culture d'une souche de *Ruminococcus gnavus* ou d'un organisme hôte transformé selon l'invention dans des conditions d'induction de l'expression du peptide, et
b) récupération du surnageant de culture comprenant le peptide.

La séparation du peptide à partir du surnageant de culture peut être réalisée par la charge, la taille et/ou le caractère hydrophobe. L'homme du métier connaît les différentes techniques permettant la séparation en fonction de la charge, de la taille et/ou du caractère hydrophobe des différents constituants d'un milieu.

Ce surnageant de culture ou moût de fermentation peut ensuite être concentré ou lyophilisé pour la formulation d'un additif alimentaire ou d'un aliment pour animaux. Le procédé peut comprendre des étapes supplémentaires de purification de la substance antimicrobienne à partir du surnageant de culture.

Si l'organisme hôte ne secrète pas la substance antimicrobienne dans le milieu de culture, une étape supplémentaire de casse des cellules et de purification de l'extrait cellulaire peut être nécessaire.

Les peptides de l'invention peuvent également être obtenus à partir du contenu caecal de rat monoxénique.

Composition

Les compositions selon l'invention comprennent un peptide selon l'invention, un organisme hôte selon l'invention, une souche selon l'invention, un moût de fermentation d'un organisme hôte selon l'invention ou un moût de fermentation d'une souche selon l'invention. Les compositions se présentent sous forme liquide ou sous forme de poudre. Ces compositions comprennent différents ingrédients.

Les compositions liquides peuvent, par exemple, comprendre un autre agent antimicrobien, par exemple de l'acide sorbique ou un sel d'acide sorbique, de l'acide benzoïque ou un sel d'acide benzoïque, de l'acide fumarique ou un sel d'acide fumarique. Les compositions de l'invention peuvent en outre comprendre du sorbitol. Le sorbitol est un agent de stabilisation et de formulation. Les compositions de l'invention peuvent également comprendre des agents anticongelants, par exemple l'éthylène glycol, le glycérol, le propylène glycol et le propane-1,2-diol.

Les compositions sous forme de poudre comprennent un support. Ce support peut être choisi parmi la farine de blé, l'amidon, la maltodextrine, le gypsum et les rafles de maïs.

Les compositions selon l'invention présentent une activité antimicrobienne. Elles fournissent des solutions alternatives à l'utilisation d'antibiotiques. Elles peuvent par exemple être utilisées dans l'élevage des animaux ou comme médicament pour l'Homme.

Les compositions de la présente invention comprennent au moins un peptide selon l'invention mais elles peuvent également comprendre d'autres substances comme des vitamines, des principes actifs autres, des acides aminés ou des sels minéraux.

Les compositions selon l'invention permettent par exemple de prévenir ou traiter l'entérite nécrotique chez les volailles ou chez les porcs et de prévenir ou traiter les maladies gastro-intestinales chez l'Homme.

Les compositions de l'invention sont, par exemple, ajoutées aux aliments pour animaux ou sont par exemple associées à une base nutritionnelle. La présente invention donc concerne aussi les aliments pour animaux comprenant une composition selon l'invention. Ces aliments se présentent habituellement sous la forme de farines ou de granulés dans lesquels sont incorporés les compositions présentant une activité antimicrobienne. La présente invention a également pour objet des aliments pour animaux comprenant un peptide selon l'invention, un organisme hôte selon l'invention ou un moût de fermentation/surnageant de culture d'un organisme hôte selon l'invention.

Par "aliment", on entend tout ce qui peut servir à la nourriture des animaux. Par "base nutritionnelle", on entend tout ce qui constitue l'essentiel de la ration alimentaire de l'animal, constitué à titre d'exemple par un mélange de céréales, de protéines et de matières grasses d'origine animale et/ou végétale. Habituellement, ces bases nutritionnelles comprennent par exemple du maïs, du blé, du pois et du soja. Ces bases nutritionnelles sont adaptées aux besoins des différentes espèces animales auxquelles elles sont destinées. Il peut par exemple s'agir de volailles (poules pondeuses, poulets de chair, dindes et canards) ou de porcs (porcs croissance et finition, porcelets).

Utilisation des peptides

La présente invention concerne également l'utilisation d'un peptide selon l'invention, d'un organisme hôte selon l'invention, d'une souche selon l'invention, d'un moût de fermentation d'un organisme hôte selon l'invention ou d'un moût de fermentation d'une souche selon l'invention pour la fabrication d'un médicament.

La présente invention concerne également l'utilisation d'un peptide selon l'invention, d'un organisme hôte selon l'invention, d'une souche selon l'invention, d'un moût de fermentation d'un organisme hôte selon l'invention ou d'un moût de fermentation d'une souche selon l'invention comme additif nutritionnel ou, en association avec d'autres composés, comme aliment pour animaux.

Selon un mode de réalisation de la présente invention, ce médicament ou cet additif nutritionnel est destiné à prévenir ou traiter l'entérite nécrotique chez les volailles ou chez les porcs.

Selon un mode de réalisation de la présente invention, ce médicament ou cet additif nutritionnel est destiné à prévenir ou traiter les maladies gastro-intestinales chez l'Homme.

**Description des figures**

Figure 1 : chromatogramme obtenu par tamisage moléculaire sur Sephadex G-75 de la fraction soluble contenue dans 10g de contenus caecaux de rats monoxéniques hébergeant la souche R. *gnavus* E1; en abscisse, volume d'élution en mL; en ordonnée, absorption à 280 nm; l'aire hachurée correspond aux fractions actives contre C. *perfringens* (fractions 325 à 358)

Figure 2A : chromatogramme obtenu par tamisage moléculaire de protéines standard sur Sephadex G-75 ; en abscisse, volume d'élution en mL; en ordonnée, absorption à 280 nm; a : albumine 67kDa ; b : ovalbumine 43 kDa ; c : chymotrypsine : 25 kDa ; d : ribonucléase A 13,7 kDa

Figure 2B : droite étalon log (MM) = f(Ve) obtenue à partir des chromatogrammes de la figure 2A ; en abscisse, volume d'élution en mL; en ordonnée, logarythme de masse moléculaire

Figure 3 : chromatogramme obtenu par HPLC sur une colonne échangeuse de cations de la fraction GF active; en ordonnée, coté gauche, absorption à 214 nm; en ordonnée, côté droit, concentration en NaCl en M; en abscisse, temps en minutes

Figure 4A : chromatogramme obtenu par HPLC en phase inverse de la fraction CM active; en ordonnée, coté gauche, absorption à 214 nm; en ordonnée, côté droit, pourcentage d'acétonitrile éluant; en abscisse, temps en minutes

Figure 4B et 4C : spectre de masse des fractions issues de l'HPLC en phase inverse; en abscisse, masse/charge; en ordonnée, intensité du signal

Figure 5 : schéma du protocole de purification de RumC à partir de contenus caecaux de rats monoxéniques hébergeant la souche R. *gnavus* E1

Figure 6 : chromatogramme obtenu par FPLC sur une résine échangeuse de cations de la fraction SN 9-17 déssalé; en abscisse, temps en minutes; en ordonnée, absorption à 214 nm

a : absorption à 214 nm
b : concentration en NaCl
c : conductivité
d : débit en mL/min

Figure 7: spectres de masse des différentes fractions au cours de la purification de la RumC; en abscisse, masse/charge; en ordonnée, intensité du signal

Figure 7A: SN 9-17

Figure 7B : fraction CM active

Figure 7C : R 10kDa

Figure 8 : spectre de masse de la fraction GF 47-50 ; en abscisse, masse/charge; en ordonnée, intensité du signal

Figure 9 : schéma du protocole de purification de RumC à partir de la souche mutante LEM 9-17

## Exemples

## Matériel et méthodes

### 1. Souches bactériennes et milieux de culture

La souche *Ruminococcus gnavus* E1, productrice de RumC, a été isolée du microbiote fécal dominant d'un adulte sain (Dabard et al., Appl. Environ. Microbiol., 67, 4111-4118, 2001).

La souche *R. gnavus* L14 est un mutant spontané de *R. gnavus* E1 ayant perdu la capacité de produire RumA *in vitro* mais synthétisant toujours RumC *in vivo.*

La souche *Ruminococcus gnavus* déposée à la CNCM le 19 décembre 2006 sous le numéro CNCM I-3705 est issue de la mutagenèse aléatoire de la souche L14, elle est capable de produire RumC *in vitro,* sur milieu gélosé supplémenté en trypsine (500 $\mu$g/ml; mais pas en milieu liquide). Il s'agit de la souche LEM 9-17.

La souche utilisée comme cible pour les tests d'activité est la souche *Clostridium perfringens* CpA (Dabard et al., Appl. Environ. Microbiol., 67, 4111-4118, 2001).

Ces quatre souches sont cultivées en anaérobiose, dans une chambre de type "Freter" en atmosphère contrôlée (85% $N_2$, 10% $H_2$, 5% $CO_2$). Elles sont incubées à 37°C dans du milieu BHI supplémenté en extrait de levure et en hémine (BHI-YH).

|  |  |
|---|---|
| BHI-YH liquide : | 37 g/L de BHI (Brain Heart Infusion, Difco Laboratories) |
|  | 5 g/L d'extrait de levure (Yeast Extract, Fluka) |
|  | 5 mg/L d'hémine (Hemin, Sigma) |
| BHI-YH gélosé : | idem + 15 g/L d'agar (BACTO™ agar, Difco Laboratories) |

### 2. Test d'activité antimicrobienne à partir d'un échantillon liquide

Pour effectuer un test d'activité antimicrobienne à partir d'un échantillon liquide, $10^4$ à $10^5$ cellules de C. *perfringens* CpA sont étalées sur milieu gélosé. Après 30 min à température ambiante, des puits de 6 mm de diamètre sont percés dans la gélose à l'aide d'une pipette pasteur. Les échantillons de volume inférieur à 100 $\mu$L sont ensuite déposés dans les puits et les boîtes sont incubées pendant 16 à 24H à 37°C. Si l'échantillon contient une substance antimicrobienne dirigée contre C. *perfringens* CpA, la croissance des bactéries est inhibée, ce qui entraîne la formation d'un "halo d'inhibition" autour du puits. Le test d'activité peut également être réalisé en déposant directement 10$\mu$L de l'échantillon à tester sur la gélose préalablement ensemencée avec C. *perfringens.*

### 3. Test d'activité antimicrobienne à partir de colonies se développant en milieu gélosé

Après 24H de culture sur milieu gélosé supplémenté ou non avec 50$\mu$g/mL de trypsine, la souche potentiellement productrice d'une substance anti-microbienne est recouverte de milieu gélosé "mou" (contenant 7,5 g d'agar/L) contenant la souche cible. Cette gélose molle est préparée en mélangeant extemporanément 5 mL de milieu gélosé en surfusion avec 5 mL de milieu liquide contenant $10^4$ à $10^5$ cellules de la souche cible. Les boîtes de pétri sont alors à nouveau incubées pendant 16 à 24H à 37°C. Un halo d'inhibition est observé autour des colonies produisant une substance anti-microbienne.

4. Elimination des débris cellulaires et résidus d'alimentation des contenus caecaux

10 g de contenu caecal sont dilués dans 30 mL de PBS (NaCl 137 mM, KCl 2,68 mM, $Na_2HPO_4$ 8,1 mM, $KH_2PO_4$ 1,47 mM, pH 7,4) puis centrifugés à 10000g et à 4°C pendant 15 min. Le culot contenant les débris cellulaires et les résidus de l'alimentation est éliminé.

5. Unités de filtration par centrifugation.

Il s'agit de membranes filtrantes permettant la séparation des protéines en deux fractions, en fonction de leur masse moléculaire après centrifugation. Ces systèmes peuvent aussi être utilisés pour concentrer ou dessaler des échantillons.

Plusieurs fabricants proposent ce genre de produit. Dans la plupart des cas les membranes sont constituées de cellulose régénérée mais il peut aussi s'agir de polyéthersulfone. Le choix du système se fait en fonction du volume des échantillons à traiter et du seuil de coupure des membranes (Tableau 1). Ces unités de filtration ont été utilisées selon les consignes fournies par le fabricant pour le choix du type de rotor et la vitesse de centrifugation. Toutefois, les temps de centrifugation ont été variables en fonction de la viscosité de l'échantillon.

Tableau 1 : Caractéristiques des unités de filtration utilisées au cours des essais de purification de RumC

| Nom | Type de membrane | Seuil de coupure | Volume maximum |
|---|---|---|---|
| AMICON® ULTRA-15 PL-5* | Cellulose régénérée | 5 kDa | 20 mL |
| MICROCON® YM-10* | Cellulose régénérée | 10 kDa | 0,5 mL |
| MICROCON® YM-3* | Cellulose régénérée | 3 kDa | 0,5 mL |
| VIVASPIN 500-5,000MWCO** | Polyethersulfone | 5 kDa | 0,6 mL |
| VIVASPIN 500-3,000MWCO** | Polyethersulfone | 3 kDa | 0,6 mL |
| * produit de la marque Millipore ; ** produit de la marque Sartorius | | | |

6. Tamisage moléculaire sur SEPHADEX G-75

Le tamisage moléculaire du surnageant de contenu caecal est réalisé sur une colonne de Sephadex G-75 (2,4 x 187 cm). L'échantillon (dont le volume est d'environ 2% du volume de la colonne) est déposé sur la colonne, l'élution étant effectuée à 4°C à un débit de 1 mL/min à l'aide de PBS et 600 fractions de 2 mL sont collectées. Un chromatogramme est obtenu par lecture de l'absorption à 280 nm de chaque fraction.

7. Tamisage moléculaire sur FPLC

Le tamisage moléculaire de la fraction CM active est réalisé sur le système FPLC. L'échantillon est chargé sur une colonne HiLoad 26/60 Superdex 30 pg (GE Healthcare). L'élution est réalisée à un débit de 2,5 mL/min avec du PBS. Le matériel protéique élué est détecté par mesure de la variation d'absorption à 280 nm. Les fractions correspondant à 2 min d'élution sont collectées automatiquement.

8. HPLC sur colonne de CM échangeuse de cations

L'HPLC échangeuse de cations est réalisée sur une chaîne WATTERS 600S CONTROLLER munie de l'injecteur WATERS 616 PUMP. L'échantillon (0,5 mL) est chargé sur une colonne semi-préparative CM-3SW SPHEROGEL de TSK. L'élution est réalisée à pH5 dans un tampon acétate de sodium 20 mM et à un débit de 0,8 mL/min, en utilisant un gradient de concentration en NaCl de 0 à 1 M. Pendant les 20 premières minutes, l'élution est effectuée en mode isocratique sans NaCl et ensuite par un gradient linéaire de concentration en NaCl de 0 à 0,5 M pendant 30 min. L'élution se poursuit alors pendant 5 min dans des conditions isocratiques, puis un saut jusqu'à une concentration de 1 M en NaCl est réalisé en 1 min, avant de poursuivre pendant 15 min dans des conditions isocratiques et, enfin, de revenir aux conditions initiales. Le matériel protéique élué est détecté par mesure de l'absorption à 280 ou 214 nm à l'aide du WATERS 486 TUNABLE ABSORBANCE DETECTOR. Les fractions sont collectées automatiquement puis dessalées à l'aide des systèmes AMICON® ULTRA PL-5.

### 9. FPLC sur colonne échangeuse de cations

Les chromatographies échangeuses de cations sont réalisées sur une chaîne FPLC. L'échantillon est chargé sur une colonne Hi-Prep 16/10 CM FF (GE Healthcare). L'élution est réalisée à pH5 dans un tampon acétate de sodium 20 mM, en utilisant un gradient de concentration en NaCl de 0 à 0,4 M. Pendant 50 minutes, l'élution est effectuée en mode isocratique sans NaCl, à un débit de 2 mL/min. L'élution se poursuit ensuite à un débit de 5 mL/min par un gradient linéaire de concentration en NaCl de 0 à 0,4 M en 40 min. Le matériel protéique élué est détecté par mesure de la variation d'absorption à 280 nm. Des fractions de 1 min sont collectées automatiquement puis dessalées sur Sep-pak®C18 Cartridges.

### 10. HPLC en phase inverse

L'HPLC en phase inverse est réalisée sur une chaîne Alliance Watters 1690 Separations Module. L'échantillon (100 $\mu$L) préalablement acidifié avec 0,1% d'acide trifluoroacétique (TFA) est chargé sur une colonne analytique Vydac 218TP52 250x2. L'élution est réalisée à débit constant (0,5 mL/min). Après 10 min d'élution en mode isocratique à 15% d'acétonitrile, un gradient linéaire de 15% à 30 % d'acétonitrile est appliqué en 60 min. Puis un saut à 70% d'acétonitrile est réalisé en 1 min et l'élution est poursuivie pendant 19 min dans des conditions isocratiques, avant de revenir aux conditions initiales. Le matériel protéique élué est détecté par mesure de la variation d'absorption à 214 nm à l'aide du Waters 996 Photodiode Array Detector. Des fractions de 0,5 mL sont collectées automatiquement. L'acétonitrile est évaporé à l'aide d'un Speed Vac Concentrator (Savant).

### 11. Spectrométrie de masse

Les analyses de spectrométrie de masse ont été réalisées au plateau protéomique Timone (Faculté de pharmacie, Marseille, France).

Les spectres de masse de type Maldi ont été obtenus sur un spectromètre ETTAN MALDI-TOF PRO (GE Healthcare,-Uppsala, SUEDE) utilisé en mode linéaire positif avec extraction retardée. Les échantillons sont co-cristallisés avec une solution de 5mg/mL d'acide $\alpha$-cyano-4-hydroxycinnamique sur la cible du Maldi par la méthode de la goutte sèche. Les spectres sont acquis avec un potentiel d'accélération de 20KV et une puissance laser réglée au niveau minimum permettant d'obtenir un bon signal. La calibration des spectres est obtenue en mode externe par acquisition d'un mélange de peptides standard de masses connues (Pepmix4, Laserbiolabs, Nice, France).

Pour certains échantillons difficiles à cristalliser, la co-cristallisation est réalisée à l'aide d'une solution d'acide 2,5 dihydroxy-benzoïque.

### 12. Séquençage d'Edman

Cette technique est basée sur la réaction d'un groupement amine terminal libre d'une protéine avec l'isothiocyanate de phényle ($C_6H_5N=C=S$). Ce composé cyclique effectue une attaque nucléophile en milieu basique sur le premier résidu acide aminé de la protéine. Le dérivé phénylthiocarbamyl (PTC) du peptide est ensuite clivé par hydrolyse. On obtient l'anilino-thiazolinone (ATZ) du premier acide aminé et la protéine ayant perdu cet acide aminé. L'ATZ-acide aminé est ensuite converti en phénylthiohydantoïne (PTH) acide aminé.

Les PTH-acides aminés obtenus successivement sont séparés et identifiés par RP-HPLC en mesurant l'absorption à 280nm et en comparant les temps d'élution.

Le cycle de réaction peut être répété et conduit ainsi à la séquence de la protéine.

### 13. Mutagenèse aléatoire

Une culture de 14mL de la souche L14 incubée pendant 24H à 37°C est répartie dans 7 tubes de 2mL puis les tubes sont centrifugés à 5800g pendant 10 min. Les culots cellulaires sont lavés, à deux reprises, avec 2mL de tampon citrate 0,1M (acide citrique 21 mg/mL, NaOH 8,8mg/mL, pH5,5) puis repris dans du tampon citrate contenant des concentrations croissantes en N-methyl-N'-nitro-N-nitrosoguanidine (NG), agent mutagène puissant.

Tableau 2 : Volumes des solutions utilisées lors des dessalages sur SEP-PAK

| Tubes | 1(T-) | 2 | 3 | 4 | 5 | 6 | 7 |
|---|---|---|---|---|---|---|---|
| [NG] finale en $\mu$g/mL | 0 | 25 | 50 | 100 | 200 | 400 | 1000 |

Les tubes sont ensuite incubés pendant 30 min à 37°C avant d'être centrifugés 10min à 5800g. Le culot est lavé avec du tampon phosphate 0,1M (KH$_2$PO$_4$ 13,6mg/mL, NaOH 2,32mg/mL, pH7) puis repris dans du milieu BHI-YH liquide.

### 14. Dessalage sur SEP-PAK®C18 Cartridges (Waters)

Selon les modèles, ces colonnes se présentent sous forme de seringues dans lesquelles la phase est coulée, ou de mini-colonnes fixées au bout d'une seringue. Le débit des tampons est assuré par une pompe péristaltique. Dans un premier temps, la colonne est équilibrée par passage d'H$_2$O puis de CH$_3$CN et enfin d'H$_2$O contenant 0,05% de TFA. L'échantillon préalablement acidifié avec 0,05% de TFA est ensuite déposé. L'élution est réalisée en 3 étapes, par ajouts successifs d'H$_2$O - TFA 0,05%, de CH$_3$CN 40% - TFA 0,05% et de CH$_3$CN - TFA 0,05%.
Trois fractions sont donc collectées : la fraction correspondant aux protéines non retenues par la colonne et aux sels (fraction NR), la fraction contenant les protéines éluées avec 40% de CH$_3$CN (fraction 40% ACN) et la fraction contenant les protéines éluées avec 100% de CH$_3$CN (fraction 100% ACN). Le solvant est évaporé à l'aide d'un Speed Vac Concentrator ou d'un Rotavapor selon le volume.
Le tableau 3 indique les volumes de chaque solution utilisée pour le dessalage de fractions issues de la purification de RumC (cf. Résultats et discussion). Ces volumes dépendent du volume de phase du SEP-PAK.

## Tableau 3 : Volumes des solutions utilisées lors de SEP-PAK en fonction des applications

| | | Dessalage de : | |
|---|---|---|---|
| Volume de : | | SN 9-17 | Fraction CM active |
| | phase | 12 mL | 1 mL |
| Equilibrage | H$_2$O | 20 mL | 5 mL |
| Equilibrage | CH$_3$CN | 20 mL | 5 mL |
| Equilibrage | H$_2$O - TFA 0,05% | 30 mL | 5 mL |
| Elution | Echantillon | 30-35 mL | 50 mL |
| Elution | H$_2$O - TFA 0,05% | 30 -35 mL | 15 mL |

| | | | |
|---|---|---|---|
| | CH$_3$CN 40% - TFA 0,05% | 20 mL | 6 mL |
| | CH$_3$CN - TFA 0,05% | 20 mL | 4 mL |

### 15. Test de résistance à la température

Des parties aliquotes d'une fraction contenant RumC sont chauffées, chacune à une température différente, à l'aide d'un bloc chauffant pendant 5 ou 15 min, puis conservées à 4°C. Elles sont ensuite soumises à un test d'activité antimicrobienne.

### 16. Test de résistance au pH

Des parties aliquotes d'une fraction contenant RumC sont diluées environ 10 fois dans le tampon désiré. Après 10 min à température ambiante, chaque solution est déposée sur une unité de filtration Vivaspin 500 3kDa puis centrifugée selon les consignes du fabricant. Du tampon est ajouté à la fraction retenue par le Vivaspin 500 puis l'unité de filtration est de nouveau centrifugée. Le volume de chacune des fractions retenues par le Vivaspin 500 3 kDa, c'est-à-dire contenant RumC, est ajusté à l'aide de tampon pour correspondre au volume de départ. Les différentes fractions sont ensuite conservées à 4°C avant d'être soumises à un test d'activité antimicrobienne.

Les filtres des Vivaspin 500 3 kDa ne supportent pas des pH supérieurs à 9. Seuls des tampons de pH acide ou neutre ont donc été testés. Leur composition est la suivante :

Tampon pH2 : KCl 50 mM - HCl 13mM
Tampon pH4,4 : tampon acétate de sodium 0,2 MpH4,4
Tampon pH7 : $KH_2PO_4$ 50 mM - NaOH 39 mM

## Résultats et discussion

### 1. Purification de RumC à partir de contenus caecaux

Bien que la souche *Ruminococcus gnavus* E1 soit cultivable, RumC n'est pas produite *in vitro* dans les conditions de culture testées. Pour purifier RumC, il faut donc travailler à partir de contenus caecaux de rats monoxéniques hébergeant la souche E1 ou créer une souche mutante de R. *gnavus* capable de produire RumC *in vitro* (voir partie 2).

#### 1.1. Dilution du contenu caecal de rat monoxénique

La première étape a consisté à diluer le contenu caecal dans du PBS.

#### 1.2. Elimination des débris cellulaires

Au cours de la seconde étape de purification les bactéries, les débris cellulaires et les résidus alimentaires sont éliminés par centrifugation du contenu caecal.

#### 1.3. Concentration

La solution est ensuite concentrée sur système AMICON® ULTRA-15 PL-5 avant d'être déposée sur une colonne de tamisage moléculaire.

#### 1.4. Test d'activité antimicrobienne

Toutes les fractions obtenues ont été testées sur la souche C. *perfringens* CpA, ce qui a confirmé la présence d'une substance anti-C. *perfringens* dans les contenus caecaux de rats monoxéniques.

Un contrôle négatif a été réalisé avec des contenus caecaux de rats axéniques (sans germe). Aucune activité anti-C. *perfringens* n'a été détectée. La substance anti-C. *perfringens* présente dans les contenus caecaux de rats monoxéniques est donc bien spécifique de R. gnavus E1.

Par la suite, chaque essai de purification a été suivi par des tests d'activité anti-*C. perfringens.* Dans un premier temps, seule la présence ou l'absence de l'activité était recherchée en s'assurant que les résultats négatifs n'étaient pas liés au seuil de sensibilité de la technique. Des tests quantitatifs ont été réalisés dans un deuxième temps, lorsque le protocole définitif a été mis au point.

#### 1.5. Tamisage moléculaire sur une colonne de SEPHADEX G-75

Un tamisage moléculaire a été réalisé sur une colonne de SEPHADEX G-75 (2,4 x 187 cm). L'élution a été suivie par lecture de l'absorption à 280 nm de chacune des 600 fractions de 2 mL collectées.

Des tests d'activité antimicrobienne ont été réalisés sur les fractions préalablement concentrées à l'aide de MICROCON® YM-3.

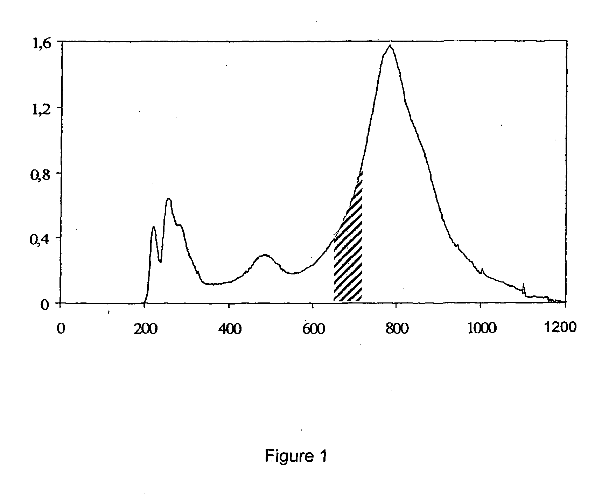

Les fractions 325 à 358 se sont avérées actives contre C. *perfringens* et ont été regroupées pour former la nouvelle fraction GF active (aire hachurée de la figure 1).

Un contrôle réalisé avec des contenus caecaux de rats axéniques a conduit à un profil d'élution comparable à celui obtenu avec les contenus caecaux de rats monoxéniques, mais aucune activité anti-C. *perfringens* n'a pu être détectée.

1.6. Evaluation de la taille de RumC

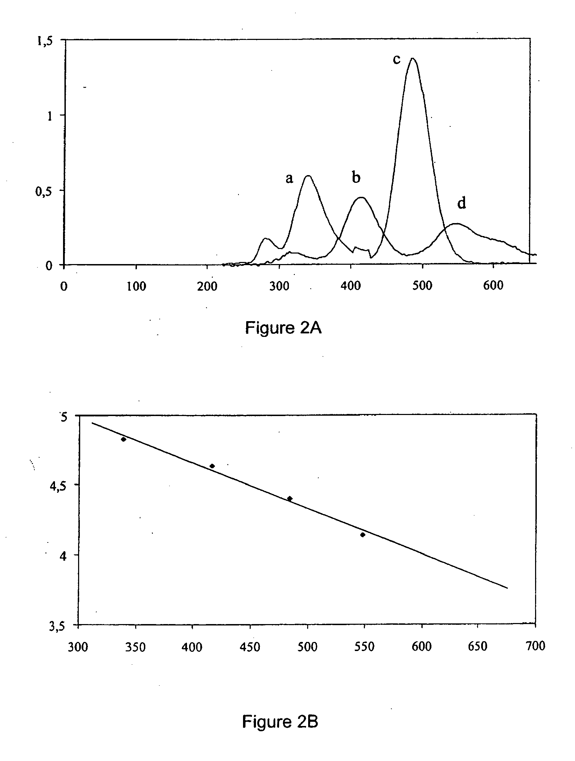

Afin d'évaluer la taille de RumC présente dans la fraction GF active, des protéines standards de masses moléculaires connues ont été déposées sur la même colonne de Sephadex G-75 et les volumes d'élution respectifs ont été mesurés (figure 2A). Une droite étalon définissant la proportionnalité entre le logarithme de la masse moléculaire (log (MM)) d'une substance et son volume d'élution (Ve) a alors été tracée (figure 2B). Avec un volume d'élution moyen de 683 mL, la substance antimicrobienne n'est pas comprise dans la gamme des protéines standards choisies, mais en extrapolant, sa masse moléculaire a pu être évaluée approximativement à 5200 Da.

1.7. Chromatographie sur résine échangeuse de cations

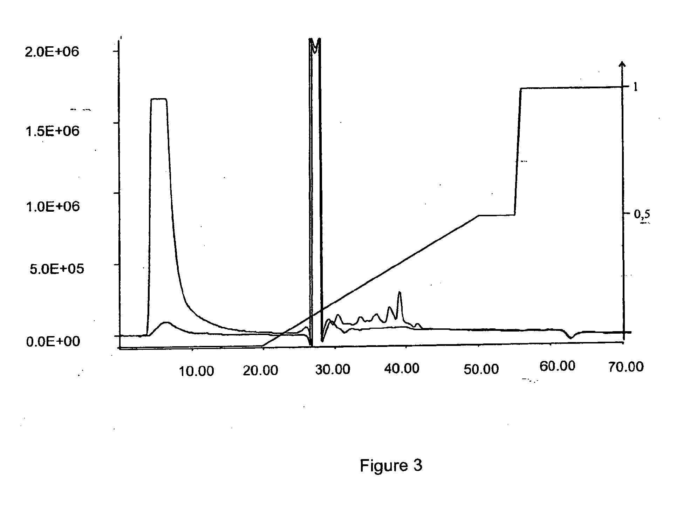

A partir de la fraction GF active, une chromatographie échangeuse de cations sur colonne de carboxyméthyl (CM) - SPHEROGEL™ a été envisagée à l'aide d'un système HPLC. L'élution a été faite par un gradient de concentration en NaCl, à pH5 et suivie à 214 nm (figure 3).

L'activité est retrouvée entre les minutes 32 et 38 (fraction CM active), ce qui correspond à une concentration en NaCl de 0,2 à 0,3M. Cette technique a permis d'éliminer des contaminants dans la fraction non retenue, notamment tous les pigments jaunes.

L'analyse par spectrométrie de masse de la fraction CM active montre qu'elle contient un peptide unique d'environ 4200 Da (résultat non montré).

Cette fraction a été soumise à un séquençage N-terminal. Les onze premiers acides aminés ont ainsi pu être déterminés : AGVIX(N/S)GTXAV (SEQ ID No. 10). Cette séquence ne présente aucune homologie forte avec des protéines connues.

Le séquençage a également mis en évidence deux séquences minoritaires.

1.8. Chromatographie en phase inverse par HPLC

Différents pics d'absorption à 214 nm ont été obtenus.

L'activité a été mise en évidence dans les 2 fractions désignées par a (ou "double pic") et b (ou "pic isolé") (Figure 4A). L'analyse par spectrométrie de masse de ces 2 fractions a révélé la présence de 3 peptides majoritaires dont les masses moléculaires respectives sont comprises entre 4230 et 4460 Da (Figure 4B et 4C).

La purification de RumC à partir de contenus caecaux a été une nouvelle fois réalisée en utilisant les 3 chromatographies mises au point, c'est-à-dire tamisage moléculaire, échangeuse de cations et phase inverse. Les profils d'élution obtenus sont comparables à ceux des premiers essais de mise au point. La méthode de purification de RumC est donc reproductible et peut être ainsi adoptée.

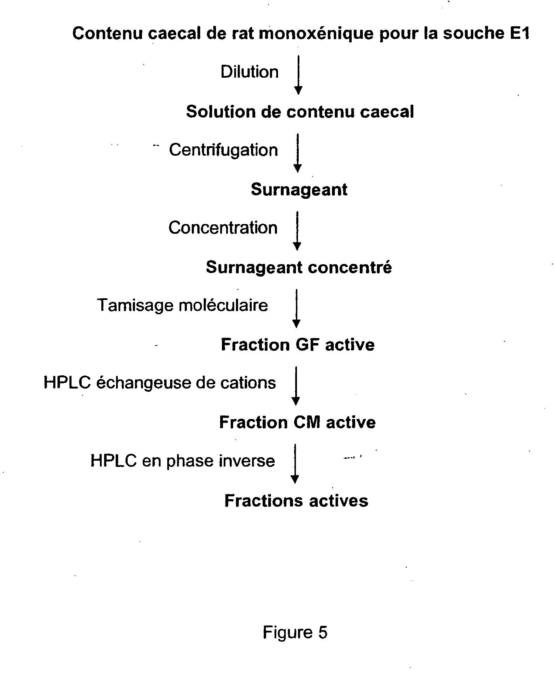

Le schéma récapitulatif de la purification de RumC à partir de contenus caecaux est représenté Figure 5.

1.9. Rendements et facteurs de purification

Par convention, on définit le nombre d'unités arbitraires d'activité (UAA) présentes dans une solution comme étant l'inverse de la dernière dilution active. Un test d'activité anti-C. *perfringens* a donc été réalisé sur des dilutions en cascade (de 2 en 2) de chaque fraction active issue de la purification.

Le facteur de purification est obtenu en comparant les activités spécifiques, exprimées en UAA / mg de protéines, de chaque fraction. Une estimation de la quantité de protéines dans les différentes fractions a été déduite de la mesure de leur absorption à 280 nm en utilisant un coefficient d'extinction massique $E^{0,1\%}$ de 1,8.

Le Tableau 4 donne les rendements en activité et les facteurs de purification des étapes du protocole de purification de RumC à partir des contenus caecaux de rats monoxéniques.

Tableau 4 : Rendements en activité et facteurs de purification lors de la préparation de RumC "in vivo" à partir de 10g de contenus caecaux de rats monoxéniques

| Fraction | V (mL) | A. anti-CpA (UAA) | m$_{protéines}$ (mg) | AS (UAA/mg) | Rdt | F purif |
|---|---|---|---|---|---|---|
| Homogénat | 39 | 780 | nd | | 100 % | |
| SN | 32 | 375 | 375 | 1 | 48 % | 1 |
| SN concentré | 14 | 350 | 270 | 1,3 | 45 % | 1,3 |
| GF active | 66 | 205 | 20 | 10,3 | 26% | 10 |
| CM active | 1-1,5 | 170 | 0,135 | 1260 | 22% | 1200 |
| Double pic | 1-1,5 | 50 | nd | | 13% | |
| Pic isolé | 1-1,5 | 50 | nd | | | |

nd = non déterminé V : volume total de la fraction en mL A. anti-CpA : activité anti-C. perfringens c'est-à-dire nombre total d'unités d'activité contenues dans la fraction m$_{protéines}$ : masse de protéines dans la fraction exprimée en mg AS : activité anti-C. perfringens spécifique en UAA/mg

$$AS = A.\ anti\text{-}CpA\ /\ m_{protéines}$$

Rdt : rendement en activité de la purification

$$Rdt = 100 \times A.\ anti\text{-}CpA\ _{Fraction\ étudiée}\ /\ A.\ anti\text{-}CpA\ _{Homogénat}$$

F purif : facteur de purification

$$F\ purif = AS\ _{Fraction\ étudiée}\ /\ AS\ _{SN}$$

1.10. Détermination de la séquence peptidique

Bien que les masses moléculaires des produits présents dans les fractions "double pic" et "pic isolé" soient différentes, le séquençage selon Edman a permis d'établir une seule séquence N-terminale majoritaire : AGXIXSGSVAV (SEQ ID No. 3).

Dans les 2 fractions concernées, une séquence minoritaire de 17 résidus a également été mise en évidence : AGPAYXVGYXGNNGAVT(SEQ ID No. 2).

2. Création d'une souche mutante par mutagenèse aléatoire

La technique retenue a été une mutagenèse aléatoire de la souche R. *gnavus* L14. Cette souche est un mutant spontané de R. *gnavus* E1 ayant perdu la capacité de produire RumA *in vitro* mais synthétisant toujours RumC *in vivo.* Cette souche a été exposée à un agent alkylant puissant, le N-méthyl-N'-nitro-N-nitrosoguanidine (NG).

Les parties aliquotes d'une même culture de la souche L14 ont ainsi été exposées à des concentrations en NG croissantes (0 à 1000 μg/mL). Après traitement, les différentes suspensions cellulaires ont été diluées puis étalées sur boîtes de pétri. Le dénombrement des colonies après culture a permis d'estimer la concentration des cellules vivantes dans les différentes suspensions.

Par comparaison avec la concentration du témoin (tube 1, sans traitement NG), un taux de mortalité a pu être déterminé pour chaque traitement. Il semble que le traitement avec la NG ait été efficace : les taux de mortalité sont compris entre 50 et 99% et augmentent avec la concentration.

Afin de rechercher le ou les mutants d'intérêt, près de 860 colonies sélectionnées au hasard parmi les clones ayant survécu aux différents traitements, ont été repiquées sur milieu gélosé supplémenté en trypsine puis soumises à un test d'activité anti-C. *perfringens.*

Quatre-vingt trois clones se sont révélés potentiellement producteurs d'une substance anti-C. *perfringens* sur milieu gélosé en présence de trypsine. Parmi eux, 20 ont été sélectionnés pour une caractérisation plus poussée.

Dans un premier temps, il a été vérifié que les mutations affectant les clones n'avaient pas restauré l'organisation chromosomique au niveau de *rumA,* par le biais d'une PCR.

Les 20 clones sélectionnés ont ensuite subi des tests d'activité anti-*Clostridium perfringens* sur milieu gélosé et en milieu liquide, en présence ou en l'absence de trypsine.

Sur milieu gélosé supplémenté en trypsine, un halo d'inhibition est présent autour de 7 des 20 clones testés.

Par contre, sur milieu gélosé sans trypsine, aucun clone ne produit de substance anti-C. *perfringens :* la trypsine est donc toujours indispensable à la synthèse de la bactériocine.

Aucune substance anti-C. *perfringens* n'est produite par les clones cultivés en milieu liquide, même en présence de trypsine.

3. Purification à partir de la souche mutante.

Des essais de production et de purification ont été réalisés sur un des 7 clones producteurs : le mutant 9-17.

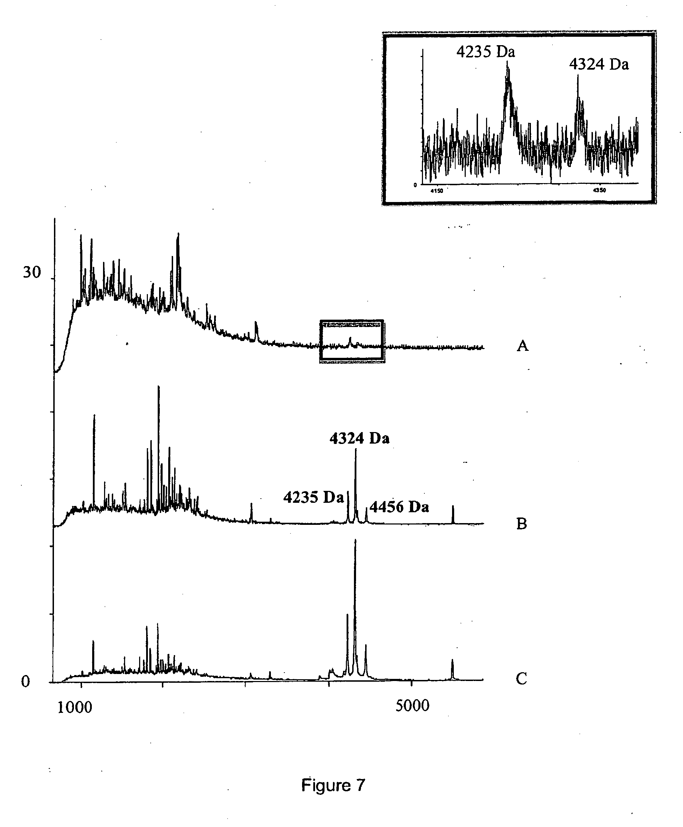

Les essais ont été réalisés sur gélose molle avec des concentrations variables en cellules et en trypsine. Après culture du mutant, la gélose a été broyée puis centrifugée pour récupérer un surnageant (SN 9-17). Ce surnageant a alors été testé contre C. *perfringens* et s'est avéré actif. Une analyse par spectrométrie de masse a été réalisée sur ce surnageant actif (Figure 7A).

3.1. Chromatographie sur résine échangeuse de cations à l'aide du système FPLC

Avant de réaliser la chromatographie échangeuse d'ions, il faut dessaler l'échantillon.

Les premiers essais ont montré qu'un dessalage du SN 9-17 sur AMICON n'était pas suffisant. Un dessalage de l'échantillon sur SEP-PAK est donc à envisager comme étape préliminaire de purification.

L'élution des protéines du SEP-PAK est réalisée avec une solution d'acétonitrile à 40 %. Après évaporation de l'acétonitrile au rotavapor, la solution est équilibrée à pH5 en la diluant dans du tampon acétate de sodium 20 mM - pH5. A partir de 65 mL de SN 9-17, on obtient 50 mL de SN 9-17 dessalé équilibré à pH5 qui sont chargés sur une colonne de carboxyméthyl-Sepharose, échangeuse de cations.

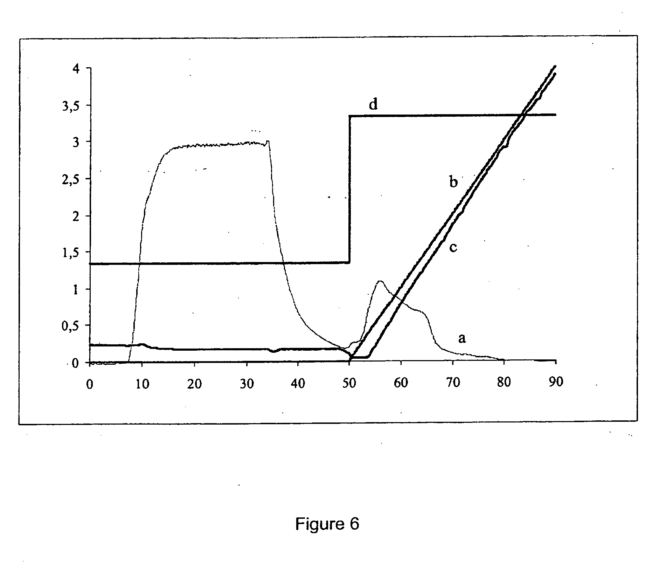

Le gradient de concentration de 0 à 0,4 M en NaCl a été effectué en 40 minutes. L'activité anti-C. *perfringens* est détectée dans les fractions éluées avec 0,2 à 0,3 M en NaCl, bien que très peu de matériel protéique ne soit présent dans ces fractions si l'on se réfère à la variation d'absorption à 280 nm (Figure 6). Ceci avait déjà été observé lors de la purification de RumC à partir de contenus caecaux. Les fractions actives ont été regroupées puis dessalées et concentrées sur SEP-PAK. L'analyse par spectrométrie de masse de cette fraction ("fraction CM active") confirme le gain de purification important apporté par la chromatographie échangeuse de cations et montre que les contaminants ont tous une masse moléculaire inférieure à 2500 Da (Figure 7B).

3.2. Tamisage moléculaire de la fraction CM

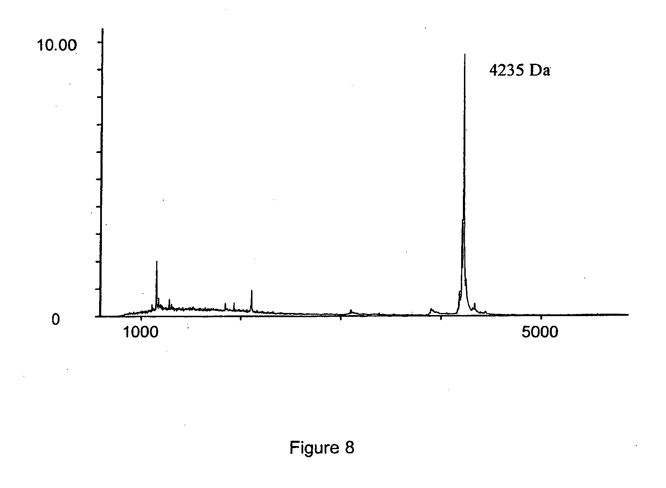

Le tamisage moléculaire de la fraction CM active a été réalisé grâce au système FPLC sur une colonne permettant la séparation de peptides de masse inférieure à 10 kDa. L'élution est suivie par lecture de la variation d'absorption à 280 nm, la quantité de matériel protéique est faible et la séparation s'avère peu satisfaisante (non montré). L'activité anti-C. *perfringens* a tout de même été testée dans les fractions collectées. Seule 5% de l'activité chargée est retrouvée après le tamisage. Cette technique n'a donc pas été retenue pour la purification. En revanche, la fraction active ("GF 47-50") a été analysée par spectrométrie de masse et s'est révélée quasi-pure avec un peptide de 4235 Da (Figure 8).

3.3. Filtration de la fraction CM

Une autre tentative pour éliminer les contaminants de la fraction CM active a été entreprise à l'aide d'unités de filtration. Comme RumC est retenue par les filtres de 5 kDa (malgré une masse moléculaire légèrement inférieure), un premier essai a été réalisé avec une unité de filtration VIVASPIN 500 dont le seuil de coupure est 5 kDa. Malheureusement, les impuretés sont elles aussi retenues par le filtre bien que leurs masses moléculaires soient inférieures à 2,5 kDa. En effet, l'analyse par spectrométrie de masse ne révèle aucun gain de purification (résultats non montrés). Un deuxième essai a donc été tenté avec une unité de filtration MICROCON dont le seuil de coupure est 10 kDa. En effet, malgré sa masse moléculaire, on a observé précédemment que RumC pouvait être retenue par des filtres de 10 kDa ("fraction R 10kDa"). L'analyse par spectrométrie de masse confirme les résultats antérieurs et permet de montrer qu'une grande partie des contaminants est éliminée dans la fraction non retenue (Figure 7C).

3.4. Chromatographie en phase inverse sur système HPLC de la fraction R 10kDa

La fraction R 10 kDa contenant les 3 pics RumC (4235 Da, 4324 Da et 4456 Da) a ensuite été soumise a une HPLC en phase inverse. Les conditions de débit et de gradient sont identiques à celles mises au point pour la purification de RumC à partir de contenus caecaux, le résultat obtenu est comparable : 2 fractions différentes sont actives contre C. *perfringens,* une fraction "double pic" et une fraction "pic isolé".

3.5. Rendements et facteurs de purification

Pour évaluer le protocole de purification, des tests d'activité quantitatifs ont été réalisés en utilisant des dilutions en cascade de 2 en 2 de chaque fraction active. Le nombre d'unités arbitraires d'activité (UAA) contenues dans chaque fraction a ensuite été calculé et le rendement de la purification a été déduit (Tableau 5). On observe une perte importante de la substance active au cours des étapes de dessalage du surnageant de culture, de la chromatographie échangeuse de cations suivie d'un autre dessalage. Il est vraisemblable que la perte ait eu lieu au cours de la chromatographie échangeuse de cations mais cela ne remet pas en cause la validité du protocole.

**Tableau 5 : Rendements de la purification de RumC à partir du surnageant de culture du mutant 9-17.**

| Fraction | V (mL) | A. anti-CpA (UAA) | Rendement |
|---|---|---|---|
| SN 9-17 | 125 | 1453 | 100 % |
| CM active | $\approx 3$ | 300 | 21 % |
| Double pic | 0,42 | 140 | 19 % |
| Pic isolé | 0,42 | 140 | |

V : volume total de la fraction en mL.

A. anti-CpA (activité anti-*C. perfringens*) ; nombre total d'unités d'activité contenues dans la fraction en UAA.

Rendement = 100 x A. anti-CpA $_{\text{Fraction étudiée}}$ / A. anti-CpA$_{\text{SN9-17}}$ ; exprimé en %.

Un autre critère important pour évaluer un protocole de purification est la mise en évidence du gain de purification apporté par chaque étape. Le facteur de purification est déterminé en comparant les activités spécifiques (UAA / mg de protéines) à chaque étape, mais pour cela il faut mesurer la masse de protéines contenu dans chaque fraction. Le suivi de purification a été réalisé par spectrométrie de masse en raison de la sensibilité de cette méthode permettant de ne pas "consommer" trop de matériel biologique. Bien que cette méthode ne soit pas quantitative, le gain de purification a été mis en évidence de manière très nette.

3.6. Spectrométrie de masse

Les fractions "double pic" et "pic isolé" ont été analysées par spectrométrie de masse. Les résultats obtenus sont identiques à ceux obtenus avec les fractions issues de la purification à partir de contenus caecaux : la fraction "double pic contient les peptides de 4324 Da et 4456 Da alors que la fraction "pic isolé" contient le peptide de 4235 Da.

3.7. Séquençage

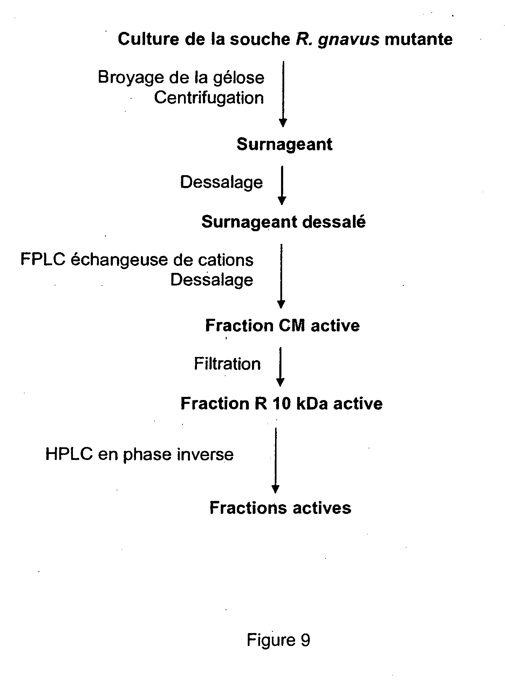

Ces fractions ont donc été séquencées.
Pour la fraction "pic isolé", la séquence obtenue est identique à la séquence déjà déterminée : AGXIXSGSVAV (SEQ ID No. 3). Une séquence minoritaire apparaît au 5ème cycle : AGPAY (SEQ ID No. 11).
En ce qui concerne la fraction "double pic", on note de légères différences : AGXVXSGSTAV (SEQ ID No. 12). La séquence minoritaire est identique : AGPAY (SEQ ID No. 11).
Le schéma récapitulatif pour la purification de RumC à partir de la souche mutante est représenté à la figure 9.
L'obtention de RumC purifié est grandement simplifiée.

4. Caractérisation des peptides RumC

4.1. Activité antimicrobienne à l'égard de différentes souches

L'activité antimicrobienne de RumC a été testée contre différentes souches pathogènes à Gram + et à Gram -, en utilisant dans un premier temps du surnageant concentré de contenu caecal de rat monoxénique pour la souche *R. gnavus* E1 (SN CCE1). D'autres tests ont ensuite été réalisés avec les 2 fractions purifiées contre les souches sensibles au SN CCE1. Les résultats sont présentés dans le tableau 6.

Tableau 6 : Résultats des tests d'activité de RumC contre différentes souches pathogènes

|  |  | Activité antimicrobienne | | |
|---|---|---|---|---|
|  |  | (taille du halo d'inhibition) | | |
|  | Souche testée / Fraction | SN CCE1 | Double pic | Pic isolé |
| Gram + | *Clostridium perfringens* CpA | + (2mm) | + (1mm) | + (1mm) |
|  | *Clostridium perfringens* S40 | + (2mm) | + (1mm) | + (1mm) |
|  | *Clostridium sporogenes* CIP 79-3 | - | nd | nd |
|  | *Clostridium acetobutylicum* BL75-41 | - | nd | nd |
|  | *Bacillus cereus* TZ 415 | + (2mm) | + (5mm) | + (3mm) |
|  | *Bacillus cereus* K 1231 | - | nd | nd |
|  | *Bacillus cereus* Z 421 | - | nd | nd |
|  | *Listeria monocytogenes* EGDE | + (1mm) | + (1mm) | + (1mm) |
|  | *Listeria monocytogenes* Scott A | - | nd | nd |
|  | *Listeria inocua* CIP 80-12 | + (1mm) | - | - |
| Gram - | *Salmonella enteridis* CIP 82-17 | + (5mm) | - | - |
|  | *Campylobacter jejuni* | - | nd | nd |

Toutes les souches *Clostridium perfringens* testées sont sensibles au peptide RumC.

4.2. Pouvoir antimicrobien contre C. perfringens

Le pouvoir antimicrobien de RumC a également été évalué en estimant la concentration minimale inhibitrice de chaque fraction purifiée RumC *"in vivo"* et en la comparant avec celle du métronidazole, antibiotique de référence utilisé contre C. *perfringens.*

Pour cela, des dilutions en cascade de 2 en 2 des fractions "double pic" et "pic isolé" ont été préparées (jusqu'à la dilution 1/512). Des tests d'activité anti-C. *perfringens* ont été réalisés à partir de ces différentes dilutions ainsi que de solutions de métronidazole à différentes concentrations (résultats non montrés).

D'après ce test, la concentration minimale inhibitrice du métronidazole est de 25 μg/mL. Ce résultat est en accord avec les résultats obtenus précédemment par Dabard *et al.* (Dabard et al., Appl. Environ. Microbiol., 67, 4111-4118, 2001).

En ce qui concerne les fractions "double pic" et "pic isolé", seules les solutions concentrées (dilution 1) sont actives. Bien que la concentration de ces solutions ne soit pas connue avec précision, elle peut être estimée, grâce au séquençage d'Edman, à environ 40 μg/mL.

Le pouvoir antimicrobien de RumC contre C. *perfringens* semble donc comparable à celui du métronidazole.

4.3. Résistance à la température

Les premiers essais préliminaires de résistance de RumC à la chaleur ont été réalisés en incubant une même quantité de peptide pendant 15 min à différentes températures. Pour les 2 fractions de RumC *"in vivo"* ("double pic" et "pic isolé"), un traitement de 15 min à 75°C n'a aucun effet sur l'activité. Par contre à 100°C, le peptide est entièrement inactivé en 15 min.

Ces essais ont été complétés à l'aide d'échantillons de RumC purifiés à partir de surnageant de culture de la souche 9-17. Le temps d'incubation aux différentes températures a été de 5 min. Le temps d'incubation à 100°C a été de 15 min.

La première température testée a été 80°C. Le premier test a été effectué avec la fraction CM active, fraction pré-purifiée contenant les 3 peptides. Contrairement aux fractions "double pic" et "pic isolé", la fraction CM active résiste à un traitement de 15 min à 100°C.

La fraction GF47-50 a ensuite été testée. Cette fraction pure contenant uniquement le peptide de 4235 Da est sensible au traitement de 15 min à 100°C. Elle est en outre sensible à un traitement de 5 min à 90°C (Tableau 7).

Tableau 7 : Tableau récapitulatif des tests de résistance à la température.

| Conditions d'incubation | | RumC *"in vivo"* | | RumC *"in vitro"* | |
|---|---|---|---|---|---|
| Température | Temps | Double pic | Pic isolé | Fraction CM active | GF 47-50 |
| 50°C | 15 min | R | R | / | / |
| 75°C | 15 min | R | R | / | / |
| 80°C | 5 min | / | / | R | / |
| 85°C | 5 min | / | / | R | / |
| 90°C | 5 min | / | / | R | s |
| 95°C | 5 min | / | / | R | / |
| 100°C | 15 min | s | s | R | s |
| R = résistant ; s = sensible ; / = non testé | | | | | |

4.4. Résistance au pH

La résistance au pH des peptides présents dans la fraction CM active a également été testée à pH 2, pH 4,4 et pH 7.

L'activité observée à pH 2 est comparable à celle observée à pH7. Après incubation de 24H à 4°C, aucune perte d'activité n'est observée à pH2.

Les peptides de la présente invention sont donc résistants au pH acide et conviennent notamment à la nutrition animale ou au domaine des médicaments.

**Revendications**

1. Peptide ayant une activité antimicrobienne contre les souches de *Clostridium perfringens* **caractérisé en ce qu'**il comprend un peptide choisi parmi les peptides suivants :

   - le peptide de la séquence SEQ ID No. 1,
   - un peptide comprenant un peptide présentant au moins 80 % d'identité avec le polypeptide de la SEQ ID No. 1,
   - le peptide de la séquence SEQ ID No. 2,
   - un peptide comprenant un peptide présentant au moins 80 % d'identité avec le polypeptide de la SEQ ID No. 2.

2. Peptide selon la revendication 1, comprenant en outre le peptide de la séquence SEQ ID No. 3.

3. Peptide selon la revendication 1, comprenant un peptide choisi parmi les peptides de séquences SEQ ID No. 4, 5 ou 6.

4. Peptide selon l'une quelconque des revendications précédentes, présentant un poids moléculaire compris entre 4000 et 4600 Da tel que déterminé par spectrométrie de masse.

5. Peptide selon l'une quelconque des revendications précédentes, isolé à partir d'une souche de *Ruminococcus gnavus* mutante.

6. Peptide selon la revendication 5, isolé à partir de la souche de *Ruminococcus gnavus* déposée à la CNCM (Collection Nationale de Cultures de Microorganismes, Institut Pasteur, 25 rue du Docteur Roux, F-75015 Paris) le 19 décembre 2006 sous le numéro CNCM I-3705.

7. Polynucléotide codant pour une activité antimicrobienne contre les souches de *Clostridium perfringens* **caractérisé en ce qu'**il est comprend un polynucléotide choisi parmi :

   - le polynucléotide selon l'une quelconque des séquences SEQ ID No. 7, 8 ou 9,
   - un polynucléotide qui s'hybride de manière sélective dans des conditions de moyenne à forte stringence au polynucléotide selon l'une quelconque des séquences SEQ ID No. 7, 8 ou 9,
   - un polynucléotide codant pour un polypeptide selon l'une quelconque des revendications 1 à 6.

8. Cassette d'expression **caractérisée en ce qu'**elle comprend dans le sens de la transcription :

   - un promoteur fonctionnel dans un organisme hôte,
   - un polynucléotide selon la revendication 7 et
   - une séquence terminatrice dans le même organisme hôte.

9. Vecteur comprenant un polynucléotide selon la revendication 7 et/ou une cassette d'expression selon la revendication 8.

10. Organisme hôte transformé avec un polynucléotide selon la revendication 7, une cassette d'expression selon la revendication 8 et/ou un vecteur selon la revendication 9.

**11.** Souche de *Ruminococcus gnavus* déposée à la CNCM (Collection Nationale de Cultures de Microorganismes, Institut Pasteur, 25 rue du Docteur Roux, F-75015 Paris) le 19 décembre 2006 sous le numéro CNCM I-3705.

**12.** Mélange protéique ou moût de fermentation susceptible d'être obtenu par un procéde de préparation comprenant les étapes suivantes :

> a) mise en culture de la souche selon la revendication 11 ou d'un organisme hôte selon la revendication 10 dans des conditions d'induction de l'expression du peptide, et
> b) récupération du surnageant de culture comprenant le peptide,
> c) optionnellement, casse des cellules et purification de l'extrait cellulaire.

**13.** Composition comprenant un peptide selon l'une quelconque des revendications 1 à 6, un organisme hôte selon la revendication 10, une souche selon la revendication 11, un moût de fermentation d'un organisme hôte selon la revendication 10 ou un moût de fermentation d'une souche selon la revendication 11.

**14.** Composition selon la revendication 13, se présentant sous forme liquide ou sous forme de poudre.

**15.** Additif nutritionnel comprenant un peptide selon l'une quelconque des revendications 1 à 6, un organisme hôte selon la revendication 10, une souche selon la revendication 11, un moût de fermentation d'un organisme hôte selon la revendication 10 ou un moût de fermentation d'une souche selon la revendication 11.

**16.** Additif nutritionnel selon la revendication 15, se présentant sous forme liquide ou sous forme de poudre.

**17.** Aliment pour animaux **caractérisé en ce qu'**il comprend une base nutritionnelle pour animaux et un additif nutritionnel selon la revendication 15 ou 16.

**18.** Utilisation d'un peptide selon l'une quelconque des revendications 1 à 6, d'un organisme hôte selon la revendication 10, d'une souche selon la revendication 11, d'un moût de fermentation d'un organisme hôte selon la revendication 10 ou d'un moût de fermentation d'une souche selon la revendication 11 pour la fabrication d'un médicament, d'un additif nutritionnel ou d'un aliment pour animaux.

**19.** Utilisation selon la revendication 18, pour la fabrication d'un médicament ou d'un additif nutritionnel destiné à prévenir ou traiter l'entérite nécrotique chez les volailles ou chez les porcs.

**20.** Utilisation selon la revendication 18, pour la fabrication d'un médicament ou d'un additif nutritionnel destiné à prévenir ou traiter les maladies gastro-intestinales chez l'Homme.

**21.** Utilisation selon la revendication 18, selon laquelle le peptide provient d'une souche endogène ou d'une souche exogène de l'animal.

**22.** Utilisation selon la revendication 18, selon laquelle le peptide provient d'une souche endogène de l'animal et selon laquelle la production du peptide par ladite souche endogène est favorisée.

**23.** Utilisation selon la revendication 18, selon laquelle le peptide provient d'une souche endogène de l'animal et selon laquelle la croissance de ladite souche endogène est favorisée.

**Patentansprüche**

**1.** Peptid mit einer antimikrobiellen Aktivität gegen die Stämme von *Clostridium perfringens,* **dadurch gekennzeichnet, dass** es ein Peptid umfasst, ausgewählt aus den folgenden Peptiden:

> - dem Peptid der Sequenz SEQ ID Nr. 1,
> - einem Peptid, umfassend ein Peptid, das mindestens zu 80 % identisch mit dem Polypeptid von SEQ ID Nr. 1 ist,
> - dem Peptid der Sequenz SEQ ID No. 2,
> - einem Peptid, umfassend ein Peptid, das mindestens zu 80 % identisch mit dem Polypeptid von SEQ ID Nr. 2 ist.

**2.** Peptid nach Anspruch 1, außerdem umfassend das Peptid der Sequenz SEQ ID Nr. 3.

**3.** Peptid nach Anspruch 1, umfassend ein Peptid, ausgewählt aus den Peptiden der Sequenzen SEQ ID Nr. 4, 5 oder 6.

**4.** Peptid nach einem der vorhergehenden Ansprüche, aufweisend ein Molekulargewicht, das zwischen 4000 und 4600 Da liegt, wie durch Massenspektrometrie bestimmt.

**5.** Peptid nach einem der vorhergehenden Ansprüche, isoliert ausgehend von einem Stamm von mutierendem *Ruminococcus gnavus.*

**6.** Peptid nach Anspruch 5, isoliert ausgehend vom Stamm von *Ruminococcus gnavus,* hinterlegt in der CNCM (Collection Nationale de Cultures de Microorganismes, Institut Pasteur, 25 rue du Docteur Roux, F-75015 Paris) am 19. Dezember 2006 unter der Nummer CNCM I-3705.

**7.** Polynukleotid, das für eine antimikrobielle Aktivität gegen die Stämme von *Clostridium perfringens* kodiert, **dadurch gekennzeichnet, dass** es ein Polynukleotid umfasst, ausgewählt aus
dem Polynukleotid nach einer der Sequenzen SEQ ID Nr. 7, 8 oder 9,

    - einem Polynukleotid, das auf selektive Weise mit mittlerer bis starker Stringenz zum Polynukleotid nach einer der Sequenzen SEQ ID Nr. 7, 8 oder 9 hybridisiert,
    - einem Polynukleotid, das für ein Polypeptid nach einem der Ansprüche 1 bis 6 kodiert.

**8.** Expressionskassette, **dadurch gekennzeichnet, dass** sie im Sinn der Transkription Folgendes umfasst:

    - einen Funktionspromoter in einem Wirtsorganismus,
    - ein Polynukleotid nach Anspruch 7, und
    - eine Terminatorsequenz im gleichen Wirtsorganismus.

**9.** Vektor, umfassend ein Polynukleotid nach Anspruch 7 und/oder eine Expressionskassette nach Anspruch 8.

**10.** Wirtsorganismus, umgewandelt mit einem Polynukleotid nach Anspruch 7, einer Expressionskassette nach Anspruch 8 und/oder einem Vektor nach Anspruch 9.

**11.** Stamm von *Ruminococcus gnavus,* hinterlegt in der CNCM (Collection Nationale de Cultures de Microorganismes, Institut Pasteur, 25 rue du Docteur Roux, F-75015 Paris) am 19. Dezember 2006 unter der Nummer CNCM I-3705.

**12.** Proteinmischung oder Fermentationsbrühe, die durch ein Zubereitungsverfahren erhalten werden kann, umfassend die folgenden Schritte:

    a) Kultivieren des Stamms nach Anspruch 11oder eines Wirtsorganismus nach Anspruch 10 unter Bedingungen der Induktion der Expression des Peptids, und
    b) Wiedergewinnen des Überstands der Kultur, umfassend das Peptid,
    c) optional, Brechen der Zellen und Reinigen des Zellextrakts.

**13.** Zusammensetzung, umfassend ein Peptid nach einem der Ansprüche 1 bis 6, einen Wirtsorganismus nach Anspruch 10, einen Stamm nach Anspruch 11, eine Fermentationsbrühe eines Wirtsorganismus nach Anspruch 10, oder eine Fermentationsbrühe eines Stamms nach Anspruch 11.

**14.** Zusammensetzung nach Anspruch 13, die eine flüssige Form oder eine Pulverform aufweist.

**15.** Nahrungsergänzung, umfassend ein Peptid nach einem der Ansprüche 1 bis 6,einen Wirtsorganismus nach Anspruch 10, einen Stamm nach Anspruch 11, eine Fermentationsbrühe eines Wirtsorganismus nach Anspruch 10, oder eine Fermentationsbrühe eines Stamms nach Anspruch 11.

**16.** Nahrungsergänzung nach Anspruch 15, die eine flüssige Form oder eine Pulverform aufweist.

**17.** Futtermittel für Tiere, **dadurch gekennzeichnet, dass** es eine Nahrungsbasis für Tiere und eine Nahrungsergänzung nach Anspruch 15 oder 16 umfasst.

**18.** Verwendung eines Peptids nach einem der Ansprüche 1 bis 6, eines Wirtsorganismus nach Anspruch 10, eines Stamms nach Anspruch 11, einer Fermentationsbrühe eines Wirtsorganismus nach Anspruch 10, oder einer Fermentationsbrühe eines Stamms nach Anspruch 11 für die Herstellung eines Medikaments, einer Nahrungsergänzung oder eines Futtermittels für Tiere.

19. Verwendung nach Anspruch 18 für die Herstellung eines Medikaments oder einer Nahrungsergänzung, ausgelegt, um der *Enteritis necrotica* bei Geflügel und bei Schweinen vorzubeugen oder sie zu behandeln.

20. Verwendung nach Anspruch 18 für die Herstellung eines Medikaments oder einer Nahrungsergänzung, ausgelegt, um den Magen-Darm-Krankheiten beim Menschen vorzubeugen oder sie zu behandeln.

21. Verwendung nach Anspruch 18 nach dem das Peptid von einem endogenen Stamm oder von einem exogenen Stamm des Tiers kommt.

22. Verwendung nach Anspruch 18 nach dem das Peptid von einem endogenen Stamm des Tiers kommt, und nach dem die Herstellung des Peptids durch den endogenen Stamm bevorzugt wird.

23. Verwendung nach Anspruch 18 nach dem das Peptid von einem endogenen Stamm des Tiers kommt, und nach dem das Wachstum des endogenen Stamms bevorzugt wird.

**Claims**

1. A peptide having an antimicrobial activity against *Clostridium perfringens* strains **characterized in that** it comprises a peptide selected from the following peptides:

   - the peptide of sequence SEQ ID No. 1,
   - a peptide comprising a peptide having at least 80% identity with the polypeptide of SEQ ID No. 1,
   - the peptide of sequence SEQ ID No. 2,
   - a peptide comprising a peptide having at least 80% identity with the polypeptide of SEQ ID No. 2.

2. The peptide according to claim 1, further comprising the peptide of sequence SEQ ID No. 3.

3. The peptide according to claim 1, comprising a peptide selected from the peptides of sequences SEQ ID No. 4, 5 or 6.

4. The peptide according to any one of the preceding claims, having a molecular weight ranging between 4000 and 4600 Da as determined by mass spectrometry.

5. The peptide according to any one of the preceding claims, isolated from *a Ruminococcus gnavus* mutant strain.

6. The peptide according to claim 5, isolated from the *Ruminococcus gnavus* strain deposited at the CNCM (Collection Nationale de Cultures de Microorganismes, Pasteur Institute, 25 Docteur Roux street, F-75015 Paris) on December 19th, 2006 under No. CNCM I-3705.

7. A polynucleotide encoding an antimicrobial activity against the *Clostridium perfringens* strains **characterized in that** it comprises a polynucleotide selected from:

   - the polynucleotide according to any one of the sequences SEQ ID No. 7, 8 or 9,
   - a polynucleotide which selectively hybridizes under medium to high stringency conditions to the polynucleotide according to any one of the sequences SEQ ID No 7, 8, or 9,
   - a polynucleotide encoding a polypeptide according to any one of claims 1 to 6.

8. An expression cassette **characterized in that** it comprises in the direction of transcription:

   - a functional promoter in a host organism,
   - a polynucleotide according to claim 7 and
   - a terminator sequence in the same host organism.

9. A vector comprising a polynucleotide according to claim 7 and/or an expression cassette according to claim 8.

10. A host organism transformed with a polynucleotide according to claim 7, an expression cassette according to claim 8 and/or a vector according to claim 9.

11. *A Ruminococcus gnavus* strain deposited at the CNCM (Collection Nationale de Cultures de Microorganismes, Pasteur Institute, 25 Docteur Roux street, F-75015 Paris) on December 19th, 2006 under No. CNCM I-3705.

12. A protein mixture or fermentation must obtainable by a preparation method comprising the following steps:

   a) culturing of the strain according to claim 11 or a host organism according to claim 10 under conditions of induction of the peptide expression, and
   b) recovering the culture supernatant comprising the peptide,
   c) optionally, disrupting the cells and purifying the cell extract.

13. A composition comprising a peptide according to any one of claims 1 to 6, a host organism according to claim 10, a strain according to claim 11, a fermentation must of a host organism according to claim 10 or a fermentation must of a strain according to claim 11.

14. The composition according to claim 13, being in a liquid or a powder form.

15. A nutritional additive comprising a peptide according to any one of claims 1 to 6, a host organism according to claim 10, a strain according to claim 11, a fermentation must of a host organism according to claim 10 or a fermentation must of a strain according to claim 11.

16. The nutritional additive according to claim 15, being in a liquid or a powder form.

17. A feedstuff **characterized in that** it comprises a nutritional base for animals and a nutritional additive according to claim 15 or 16.

18. A use of a peptide according to any one of claims 1 to 6, a host organism according to claim 10, a strain according to claim 11, a fermentation must of a host organism according to claim 10 or a fermentation must of a strain according to claim 11 for the manufacture of a drug, a nutritional additive or a feedstuff.

19. The use according to claim 18, for manufacturing a drug or a nutritional additive intended to prevent or to treat necrotic enteritis in poultry or pigs.

20. The use according to claim 18, for manufacturing a drug or a nutritional additive intended to prevent or to treat gastrointestinal diseases in humans.

21. The use according to claim 18, wherein the peptide is derived from an endogenous or exogenous strain of the animal.

22. The use according to claim 18, wherein the peptide is derived from an endogenous strain of the animal and wherein the production of the peptide by said endogenous strain is promoted.

23. The use according to claim 18, wherein the peptide is derived from an endogenous strain of the animal and wherein the growth of said endogenous strain is promoted.

Figure 1

Figure 2A

Figure 2B

Figure 3

Figure 4

**Contenu caecal de rat monoxénique pour la souche E1**

Dilution

**Solution de contenu caecal**

Centrifugation

**Surnageant**

Concentration

**Surnageant concentré**

Tamisage moléculaire

**Fraction GF active**

HPLC échangeuse de cations

**Fraction CM active**

HPLC en phase inverse

**Fractions actives**

Figure 5

Figure 6

Figure 7

Figure 8

**Culture de la souche *R. gnavus* mutante**

Broyage de la gélose
Centrifugation

**Surnageant**

Dessalage

**Surnageant dessalé**

FPLC échangeuse de cations
Dessalage

**Fraction CM active**

Filtration

**Fraction R 10 kDa active**

HPLC en phase inverse

**Fractions actives**

Figure 9

EP 2 152 881 B1

**RÉFÉRENCES CITÉES DANS LA DESCRIPTION**

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 0068401 A

**Littérature non-brevet citée dans la description**

- **ROSS et al.** *Int. J. Food Microbiol.,* 2002, vol. 79, 3-16
- **MOOTZ et al.** *Curr. Opin. Chem. Biol.,* 1997, vol. 1, 543-551
- **KEATING et al.** *Curr. Opin. Chem. Biol.,* 1999, vol. 3, 598-606
- **JACOB et al.** *Ann. Inst. Pasteur (Paris,* 1953, vol. 84, 222-224
- **LUCHANSKY.** *Antonie Van Leeuwenhoek,* 1999, vol. 76, 335
- **O'SULLIVAN et al.** *Biochimie,* 2002, vol. 84, 593-604
- **MORISSET et al.** *Appl. Environ. Microbiol.,* 2004, vol. 70, 4672-4680
- **FLYNN et al.** *Microbiol.,* 2002, vol. 148, 973-984
- **MCCORMICK et al.** *Appl. Environ. Microbiol.,* 1998, vol. 64, 4757-4766
- **GARNEAU et al.** *Appl. Environ. Microbiol.,* 2003, vol. 69, 1352-1358
- **BIET et al.** *Microbiol.,* 1998, vol. 144, 2845-2854
- **MILLER et al.** *Appl. Environ. Microbiol.,* 1998, vol. 64, 14-20
- **RICHARD et al.** *J. Bacteriol.,* 2004, vol. 186, 4276-4284
- **KLOCHE et al.** *Appl. Microbiol. Biotechnol.,* 2005, vol. 67, 532-538
- **SCHOEMAN et al.** *Yeast,* 1999, vol. 15, 647-656
- **VAN REENEN et al.** *Int. J. Food Microbiol.,* 2003, vol. 81, 29-40
- **RODRIGUEZ et al.** *Int. J. Food Microbiol.,* vol. 80, 101-116
- **SUAU et al.** *Appl. Environ. Microbiol.,* 1999, vol. 65, 4799-4807
- **HUGHES ; ROWLAND.** *Microbial Ecology Health Disease,* 2000, vol. 2, 179-185
- **BRY et al.** *Science,* 1996, vol. 273, 1380-1383
- **HOOPER et al.** *Science,* 2001, vol. 291, 881-884
- **DUCLUZEAU et al.** *Microbial Ecology and Intestinal Infections,* 1988
- **FONS et al.** *Microbial Ecology in Health and Disease,* 2000, vol. 2, 240-246
- **PETIT et al.** *Trends Microbiol.,* 1999, vol. 7, 104-110
- **MEAD et al.** *Emerg. Infect. Dis.,* 1999, vol. 5, 607-625
- **VALANCONY.** *Bulletin des GTV,* 2001, vol. 12, 9-12
- **DABARD et al.** *Appl. Environ. Microbiol.,* 2001, vol. 67, 4111-4118
- **GOMEZ et al.** *J. Bacteriol.,* 2002, vol. 184, 18-28
- **SAMBROOK ; FRISTSCH ; MANIATIS.** Molecular cloning: a laboratory manual. Cold Spring Harbor Laboratory Press, 1989
- **ROBERTS et al.** *Current Gene,* 1989, vol. 15, 177-180
- **STUDIER et al.** *Methods in enzymology,* 1990, vol. 185, 60-89
- **ELLEDGE et al.** *Proc Natl Acad Sciences, USA,* 1991, vol. 88, 1731-1735
- **TSCHOPP et al.** *Biotechnology,* 1987, vol. 5, 1305-1308
- **WATERHAM et al.** *Gene,* 1997, vol. 186, 37-44
- **MAUNDRELL.** *J. Biol. Chem.,* 1989, vol. 265, 10857-10864
- **AUSUBEL et al.** Current Protocols in Molecular Biology. Greene Publishing Associates, Inc, 1992

SEQUENCE LISTING

<110> ADISSEO FRANCE SAS

<120> Peptide RumC présentant une activité antimicrobienne

<130> xx-53845

<160> 12

<170> PatentIn version 3.2

<210> 1

<211> 17
<212> PRT
<213> Ruminococcus gnavus

<220>
<221> misc_feature
<222> (6)..(6)
<223> Xaa can be any naturally occurring amino acid

<220>
<221> misc_feature
<222> (10)..(10)
<223> Xaa can be any naturally occurring amino acid

<220>
<221> misc_feature
<222> (13)..(13)
<223> Xaa can be any naturally occurring amino acid

<220>
<221> misc_feature
<222> (15)..(15)
<223> xaa can be any naturally occurring amino acid

<400> 1

```
Ala Gly Pro Ala Tyr Xaa Val Gly Tyr Xaa Gly Asn Xaa Gly Xaa Val
1               5                   10              15

Thr
```

<210> 2
<211> 17
<212> PRT
<213> Ruminococcus gnavus

<220>
<221> misc_feature
<222> (6)..(6)
<223> Xaa can be any naturally occurring amino acid

<220>
<221> misc_feature
<222> (10)..(10)
<223> Xaa can be any naturally occurring amino acid

<400> 2

```
Ala Gly Pro Ala Tyr Xaa Val Gly Tyr Xaa Gly Asn Asn Gly Ala Val
1               5                   10              15

                                    Thr
```

<210> 3
<211> 11

<212> PRT
<213> Ruminococcus gnavus

<220>
<221> misc_feature
<222> (3)..(3)
<223> xaa can be any naturally occurring amino acid

<220>
<221> misc_feature
<222> (5)..(5)
<223> Xaa can be any naturally occurring amino acid

<400> 3

```
Ala Gly Xaa Ile Xaa Ser Gly Ser Val Ala Val
1               5               10
```

<210> 4
<211> 63
<212> PRT
<213> Ruminococcus gnavus

<400> 4

```
Met Arg Lys Ile Val Ala Gly Lys Leu Gln Thr Gly Ala Asp Phe Glu
1               5                   10                  15

Gly Ser Lys Trp Gly Cys Val Cys Ser Gly Ser Thr Ala Val Ala Asn
            20              25              30

Ser His Asn Ala Gly Pro Ala Tyr Cys Val Gly Tyr Cys Gly Asn Asn
            35              40              45

Gly Val Val Thr Arg Asn Ala Asn Ala Asn Val Ala Lys Thr Ala
        50              55              60
```

<210> 5
<211> 63
<212> PRT
<213> Ruminococcus gnavus

<400> 5

```
Met Arg Lys Ile Val Ala Gly Lys Leu Gln Thr Gly Ala Asp Phe Glu
1               5                   10                  15

Gly Ser Lys Gly Gly Cys Lys Cys Ser Gly Gly Ala Val Val Glu Asn
            20              25              30

Ser His Asn Ala Gly Pro Ala Tyr Cys Val Gly Tyr Cys Gly Asn Asn
            35              40              45
```

Gly Val Val Thr Arg Asn Ala Asn Ala Asn Leu Ala Arg Thr Lys
50 55 60

<210> 6
<211> 63
<212> PRT
<213> Ruminococcus gnavus

<400> 6

Met Lys Leu Val Glu Thr Lys Thr Thr Lys Thr Gly Thr Asn Phe Glu
1 5 10 15

Gly Asn Arg Ala Gly Cys Ile Cys Asn Gly Thr Val Ala Val Ala Asn
20 25 30

Ser His Asn Ala Gly Pro Ala Tyr Cys Val Gly Tyr Cys Gly Asn Ser
35 40 45

Gly Val Val Thr Arg Asn Ala Asn Ala Asn Val Ala Lys Thr Ala
50 55 60

<210> 7
<211> 192
<212> DNA
<213> Ruminococcus gnavus

<400> 7

```
atgagaaaaa tcgtagcagg aaagttacag acaggagcag actttgaagg cagcaaatgg      60
ggatgtgttt gtagtggaag cacagcagta gcaaactctc ataatgcagg accggcgtat     120
tgcgtaggat actgtggaaa caacggagta gtgactagaa atgctaatgc aaatgtcgca     180
aaaacggcat aa                                                          192
```

<210> 8
<211> 192
<212> DNA
<213> Ruminococcus gnavus

<400> 8

```
atgagaaaaa tcgtagcagg aaagttacag acaggagcag actttgaagg cagcaaaggt      60
ggatgtaaat gcagtggcgg tgcagtagta gaaaactctc ataatgcagg accagcgtat     120
tgcgtgggat actgtggaaa caacggagtg gtaacaagaa atgcgaatgc gaatcttgca     180
agaacaaaat aa                                                          192
```

<210> 9
<211> 192
<212> DNA

<213> Ruminococcus gnavus

<400> 9

atgaaattag tagaaacaaa aacaacaaaa acaggaacaa actttgaagg gaatagagct    60

ggatgtattt gtaatggcac tgtagcagta gcaaattctc ataatgcagg accagcatat    120

tgtgttgggt attgcggaaa tagtggagta gtaacaagaa atgcgaatgc aaatgtcgca    180

aaaacagcat aa    192

<210> 10
<211> 11
<212> PRT
<213> Ruminococcus gnavus

<220>
<221> misc_feature
<222> (5)..(5)
<223> Xaa can be any naturally occurring amino acid

<220>
<221> misc_feature
<222> (6)..(6)
<223> Xaa can be Asn or Ser

<220>
<221> misc_feature
<222> (9)..(9)
<223> Xaa can be any naturally occurring amino acid

<400> 10

```
Ala Gly Val Ile Xaa Xaa Gly Thr Xaa Ala Val
1               5               10
```

<210> 11
<211> 5
<212> PRT
<213> Ruminococcus gnavus

<400> 11

```
Ala Gly Pro Ala Tyr
1               5
```

<210> 12
<211> 11
<212> PRT
<213> Ruminococcus gnavus

<220>
<221> misc_feature
<222> (3)..(3)

<223> Xaa can be any naturally occurring amino acid

<220>
<221> misc_feature
<222> (5)..(5)
<223> Xaa can be any naturally occurring amino acid

<400> 12

```
Ala Gly Xaa Val Xaa Ser Gly Ser Thr Ala Val
1               5                   10
```